# EUROPEAN PATENT SPECIFICATION

(11) **EP 3 983 804 B1**
(45) Date of publication and mention of the grant of the patent: **06.08.2025**
(21) Application number: 20731488.1
(22) Date of filing: 12.06.2020
(51) Int. Cl.: G01N 33/543, G01N 33/569, G01N 33/58

(54) **SYNTHROCYTE: ERYTHROCYTE-MIMICKING REAGENT AND FAST METHODS FOR PATHOGEN CHARACTERIZATION AND SEROLOGY TESTING**
REAGENZIEN UND SCHNELLE VERFAHREN ZUR PATHOGENCHARAKTERISIERUNG UND SEROLOGIEUNTERSUCHUNG
RÉACTIFS ET PROCÉDÉS RAPIDES DE CARACTÉRISATION DE PATHOGÈNES ET D'EXAMENS SÉROLOGIQUES

(30) Priority: 13.06.2019 EP 19382496
(43) Date of publication of application: 20.04.2022
(73) Proprietor: Fundació Hospital Universitari Vall d'Hebron - Institut de Recerca, 08035 Barcelona (ES)
(72) Inventor: SÁNCHEZ CANO, Ana, 08191 RUBÍ (ES); ANTÓN PAGAROLAS, Andrés, 08915 BADALONA (ES); ANDRÉS VERGÉS, Cristina, 08396 SANT CEBRIÀ DE VALLALTA (ES); HERANCE CAMACHO, José Raúl, 08206 SABADELL (ES); PUMAROLA SUÑÉ, Tomás, 08023 BARCELONA (ES); BALDRICH RUBIO, Eva, 08223 TERRASSA (ES)
(74) Representative: ZBM Patents - Zea, Barlocci & Markvardsen
(86) International application number: PCT/EP2020/066292
(87) International publication number: WO 2020/249724

(56) References cited:
- EP-A1- 0 174 195
- WO-A1-92/05440
- WO-A1-95/31731
- US-A- 4 355 102
- US-A- 5 405 784
- MICROMOD: "Micro -Tools in Life Science Modular Designed Particles", 1 January 2017 (2017-01-01), pages 1 - 18, XP093093499, Retrieved from the Internet <URL:https://donar.messe.de/exhibitor/labvolution/2017/R260918/micromod-product-overview-eng-490167.pdf> [retrieved on 20231020]

## Description

### Technical Field

The present invention relates to the field of serological assays and to methods for detecting and characterizing pathogens and evaluate the immunological response against pathogen infection or vaccination. The invention also relates to consumables for classical hemagglutination (HA) and hemagglutination inhibition (HAI) assays.

### Background Art

Some pathogens, such as influenza viruses, display a high genetic diversity, which explains that these pathogens cause seasonal epidemics, local outbreaks and sporadic global pandemics. In the case of influenza viruses, the high rate of change is especially evident for hemagglutinin (HA) and neuraminidase (NA), the two main proteins of the viral envelope and the leading antigenic determinants of the virus. Continuous evolution hampers the recognition by the immune neutralizing antibodies, acquired over previous exposures to the virus by natural infection or by vaccination, and determines the virus features, including its antigenicity, tropism, transmission capacity, virulence and susceptibility to antivirals.

The main genetic mechanisms of evolution of influenza viruses are the antigenic drift, or minor variations, and the antigenic shift, or major variations. Genetic drift is caused by the high error rate of the viral polymerase during replication and its lack of proof-reading activity, which leads to the gradual accumulation of mutations and amino acid changes in the viral proteins. The annual epidemics are caused by viruses of the same subtype than those having circulated in previous seasons, but that have evolved by antigenic drift. On the other hand, the segmented nature of the influenza virus genome permits also the reassortment of the viral segments between different viruses that are co-infecting a single cell. This occurs mainly in freshwater birds, and to a lesser extent in pigs, which are hosts of a wide range of subtypes of influenza A virus. These reassortments produce larger antigenic changes, especially when the resulting strain acquires an HA displaying antigenic characteristics that are novel for the human population. While antigenic drift occurs gradually, antigenic change occurs infrequently, abruptly and unpredictably. If the resulting reassortant strain is capable of transmitting efficiently from person to person, it will be potentially pandemic.

Influenza viruses are an important cause of morbidity and mortality in the population. Apart from the human cost, annual flu epidemics entail high expenses for countries and their health systems. The severity of the seasonal epidemic is proportional to the degree of immune protection of the population, which in turn is related to the high rate of change of the virus. The control, prevention and treatment of the influenza infection are based on prophylaxis, vaccination, antiviral treatment and virological surveillance. Vaccination is the leading measure to prevent influenza infections, minimizes the risk of clinical complications and reduces their direct and indirect costs. Safe and effective vaccines have been used for more than 60 years. However, the constant evolution of influenza viruses makes permanent virological surveillance absolutely mandatory, in order to monitor the characteristics and evolution of the circulating viruses, detect early viruses with pandemic potential and reformulate annually the vaccine composition.

The antigenic characterization of influenza viruses is carried out by the hemagglutination inhibition assay (HAI), which allows comparing the antigenic characteristics of circulating viruses with those of strains included in the vaccine or other reference strains. HAI can be used for the typing and subtyping of influenza viruses, as well as for the detection and titration of specific antibodies against influenza viruses. Hemagglutination assays are based on the ability of the HA of the viral envelope to bind to cellular receptors of sialic acid in the membrane of erythrocytes, causing their agglutination (hemagglutination) and altering their sedimentation. The formation of these complexes can be inhibited by specific antibodies against HA, which block HA physically and prevent hemagglutination. Using a battery of antibodies specific for different influenza types and subtypes, HAI allows classifying unknown viral isolates. In the same way, using a battery of viral isolates of different types and subtypes allows characterizing the antibodies in a test sample, for instance, to study the immunological response of an individual after vaccination or after exposure to viral infection.

HAI has been the main technique for influenza antigenic characterization and antibody detection for decades, and it is still valid. However, the results obtained by HAI are highly variable intra- and inter-laboratory. Likewise, expertise is fundamental in the interpretation of results, which are often subjective. Another limitation is the need to eliminate non-specific HAs from the test and reference sera (mainly lectins that interfere with the assay). On the other hand, the recent adaptation of influenza A(H3N2) to human receptors and its loss of affinity for avian receptors, has forced the modification of the HAI method, including the re-selection of the erythrocytes needed and the addition of oseltamivir (an inhibitor of viral NA activity) in the procedure. Nowadays, while HAI employs avian erythrocytes (chicken or turkey) for the study of influenza B and A(H1N1) pdm09 viruses, influenza A(H3N2) viruses are studied using mammalian erythrocytes (mostly guinea pig, since obtaining human erythrocytes for this type of tests is ethically more complex). Turkey RBCs are frequently chosen for HAI testing because the settling time is shorter, inhibition patterns are clearer and the cells are readily available. However, it is known that some strains of influenza viruses, especially during initial and early passages, may react poorly to turkey RBCs. Guinea-pig RBCs have consistently been demonstrated to be more sensitive in detecting human influenza viruses and should therefore be used for maximum sensitivity when detecting newly isolated viruses. Nonetheless, the most critical drawback of HAI is that hemagglutination has to be carried on fresh avian and/or mammalian erythrocytes, depending on the type or influenza subtype under study. Fresh erythrocytes are variable, display short half-life (<2 weeks), are hard to mass-produce for their global distribution, and are difficult and expensive to obtain commercially, which places them as the main source of variability of HAI. It is worth noting that the alternative use of seroneutralization is even more complex and variable; and that substitution by methods such as ELISA, latex agglutination assays, biosensors or PCR would not provide all the information afforded by HAI.

In an attempt to solve some of the drawbacks associated with hemagglutination assays, the inventors of an international application with publication number WO201133450 (Mesoscale technologies LLC) disclosed different serology assays. The document addressed the problem of having to use fresh blood with the associated inter-laboratory variability. The main object was an array of viral receptors to provide an alternative methodology to HAI, for example for determining the efficacy of a vaccination protocol for the treatment or prevention of an infection, or to ensure that selected vaccines provide the adequate protection. In this proposed method, a sample containing the pathogen (or antigen from a virus) is placed on the array for capture. "Particle reagents" with a ligand capable to recognize the pathogen are added to form a sandwich. The invention includes also the possibility to incubate first the test sample with the particle reagents. The invention considers the possibility as well to study samples supposed to have specific antibodies to be detected, which should inhibit the binding of particle reagents to the ligand. Particular antigens of the virus are sialic receptors, and particular ligands added in the particles (particle reagent) include sialic acids. Examples of virus include influenza virus. Detection of the binding is performed either with a label, preferably sited on the particle reagent, or by direct visualization of the particles. Exemplified particle reagents include red blood cells (RBC).

On the other hand, poly(glycidyl methacrylate) blue microspheres with immobilized bovine serum albumin (BSA) were indicated to exhibit agglutination and sedimentation patterns resembling those of the conventional microplate tests in which RBC are used as carrier particles. See Hosaka et al., "Preparation of microspheres of poly(glycidyl methacrylate) and its derivatives as carriers for immobilized proteins", Immunological communications - 1980, vol. no. 12(5), pp.: 509-517. In this document, the authors synthesized blue particles that could be modified by immobilizing on their surface either an antibody or BSA. From their assays, they concluded that these particles could be used in replacement of latex particles and as possible substitutes of RBC in classical HAI assays. However, the concluding remarks by Hosaka et al. in relation with hemagglutination derive from an assay with a simpler structure, comprising bead-bound BSA and tested in front of anti-BSA antibodies only. Thus, the beads and method reported were not tested in a real scenario. On the other hand, when sedimentation rates of the poly(glycidyl methacrylate) blue microspheres were compared with fresh blood (sheep or chicken), the microspheres were free of any immobilized ligand for the recognition of pathogens. Therefore, the data reported do not allow concluding or confirming that indeed the microspheres can replace RBC in HAI assays. Finally, agglutination assays using the poly(glycidyl methacrylate) blue microspheres took 2 h, which is similar and not better than the time needed for HAI according to the authors.

Another methodology used for the detection of pathogens and/or antibodies against them is the latex agglutination test, often known as turbidimetric tests. In this case, latex nano- or micro-beads are modified with pathogen antigens (to detect antibodies against the pathogen) or with specific antibodies (to detect pathogens or pathogen antigens). In the presence of its target, beads agglutinate, changing the suspension physical properties, such as turbulence, transmittance, or absorbance. One of the main drawbacks on this classical method is its limited sensitivity, especially if the result is interpreted visually. Accordingly, these assays are generally detected spectrophotometrically. Many attempts have been made to objectivise and ameliorate the readout of this type of results. For example, in patent US4355102A of 19th October 1982 (Institute National de la Santé et de la Recherche Medicale, INSERM), the authors claim a turbidimetric method to detect neuraminidase activity. For this, latex particles 0.85 µm in diameter are modified with sialylated molecules, which act as the neuraminidase substrate. These beads are then mixed in the wells of a 96-well microtiter plate with viruses and buffer, the plate is closed with a cover, surrounded with adhesive tape, placed on a plate agitator in an incubator at 37° C for 1 hour, and then incubated for one night at room temperature. Over this time, large complexes form between the enzyme and its bead-bound substrate, which gives rise to agglutination of the spheres. As stated by the authors, the resulting bead macroagglutinates are observable with the naked eye if the plate is placed on a negative slide viewer, and against a black background, under the effect of a diffuse light source, also described in the text as an oblique light. Hence, a certain equipment is needed, including a thermostated incubator and diffuse light source, to carry out the assay and to interpret the results of the assay, which requires an overnight incubation. Consequently, a method of this type is not easier or faster to carry and interpret than classical HAI.

In a different approach, Patent US5405784A of 11th April 1995 (Chemunex), describes a method for the individual determination of the presence or absence of multiplexed ligands in a specimen using fluorescent beads (0.05-10.0 µm), modified with anti-ligands. Modified fluorescent beads and ligand-containing sample are then incubated for 2 hours at 37°C. In the presence of ligands, bead agglutination occurs, which affects the total amount of fluorescent lights and defracted lights in the mixture, allowing to measure the fluorescence per ligand to determine the number of non-agglutinated beads, the number of agglutinated beads, the number of bead aggregates and, for each aggregate, the number of beads it comprises. For this, a flow cytometer, an image analyser, or a laser sweep system has to be used. Although an improved sensitivity is attained, the need to use equipment to carry out a 2-hour assay is not a feasible alternative to classical HAI.

It is thus clear that new approaches are needed in order to overcome the above-mentioned drawbacks of the HAI assays that are used worldwide for antigenic characterization of pathogens, for the seasonal epidemic control, and for monitoring the immune protection of the population after exposure to pathogens or vaccines.

### Summary of Invention

The invention is set out in the appended claims.

The inventors have developed particles with particular material mass density, diameter and surface charge in certain buffered media, which can be functionalized to have immobilized on their surface particular antigen-recognizing ligands. Such particles sediment when introduced in a buffered aqueous sample within 1 to 20 minutes (min), or indeed in less than 10 min (from 1 to 10 min), and even from 1 to 5 minutes, displaying different sedimentation pattern when the particles are agglutinated (i.e. forming a net due to interactions of the particles with substances in the buffered aqueous sample) in relation to particles that do not agglutinate (i.e. are not forming a net). Due to the coloured feature, an easy visual detection of said differential sedimentation pattern is attained by the naked eye, without requiring any external equipment (such as lasers, fluorescence readers, or detectors or any type) for result interpretation.

The particles produced are useful for detecting the presence of pathogens (or pathogen antigen) in samples (biological samples isolated from organisms or environmental samples), or for detecting specific antibodies recognizing said pathogens (or pathogen antigen). In this context, binding of the pathogen (or pathogen antigen) to the particles modify their surface properties, and ultimately cause their agglutination. Agglutination of the particles takes place when, due to the presence of the pathogen (or pathogen antigen) in the sample or buffered media, interaction between the recognized antigen and the antigen-recognizing ligands on the surface of multiple particles occurs. This interaction results in the change of the particles' surface properties, and ultimately in the formation of a net of particles and pathogen (or pathogen antigen), with a particular behaviour (i.e. turbulence in the buffered media/isolated sample, called in this description *agglutination*). When no interaction with pathogen (or pathogen antigen) occurs, no agglutination or formation of the net occurs and the particles settle down forming a pellet in the buffered sample, usually on the bottom of the vessel where the contact with the sample was carried out. Similarly, the presence of competing antibodies inhibits antigen binding to the particles, no agglutination or formation of the net occurs, and the particles sediment forming a pellet on the bottom of the recipient. Therefore, easy visualization of the sedimentation pattern allows easy detection of agglutination or non-agglutination of the particles, without the requirement of any external equipment, which is directly dependent on the presence and/or type of particular pathogen and/or antibodies in the sample.

Thus, in a first aspect described herein are coloured particles comprising:
- a core with a surface, said core of a material with a mass density from 1.5 to 2.5 g/cc; and a diameter from 3 to 10 µm, and wherein at least the surface is coloured and it comprises one or more functional groups selected from -COO-R¹, -NR²R³ and combinations thereof, wherein R¹ is independently selected from H, and (C₁-C₈)-alkyl, and R² and R³ are independently selected from H, (C₁-C₈)-alkyl, and (C₁-C₈)-alkoxyl; and
- a pathogen (or pathogen antigen) ligand immobilized on the surface of the core.

These coloured particles comprising a surface and a pathogen (or pathogen antigen) ligand on it, are ligand-modified particles that display sedimentation in a vessel, when placed in a buffered aqueous media of pH equal or higher to the isoelectric point (Ip) of the components of the particle (i.e. immobilized pathogen ligand and charged functional groups, also called herewith average isoelectric point), forming particle sediments in a time from 1 to 20 min, indeed in 10 min (in some cases, even in less than 5 min). On the contrary, they exhibit a differential sedimentation pattern, or visibly differential behaviour, in samples comprising a pathogen (or pathogen antigen), in which they agglutinate due to the interaction of the pathogen in the sample with the pathogen ligand on the particle surface, and do not form clear sediments (i.e pellets) within the said 1-20 min, or 1-10 min incubation time, but appear as a turbulent media. This change in sedimentation is observed visually by human naked eye, without the need of any external equipment (such as lasers, fluorescence readers, or detectors or any type). The coloured particles described herein are, in other words, particles of visible colour by the naked human eye, and this is what has to be understood by "coloured" in this description.

Due to the immobilized pathogen (or pathogen antigen) ligand and also to the one or more functional groups on the surface of the particle core, the particles have a particular surface charge in a buffered aqueous media and depending on this media pH. Particles are conceived to have a net surface charge (positive or negative) in particular pH conditions.

The differential sedimentation pattern or behaviour in a buffered aqueous media is the result of the combination of particular mass densities of the particles, particular diameters of the particles and to the surface charge of the whole particle in said particular buffered aqueous media.

Thus, a functional feature of the ligand-modified particles is that they display a differential sedimentation pattern when agglutinated or non-agglutinated and when suspended in an aqueous buffered solution at a pH equal or higher than the net isoelectric point of the particle surface components. This net isoelectric point of the particle is the result of isoelectric point of the pathogen ligand immobilized on the surface and also of the charge (positive or negative) provided by one or more functional groups on the surface.

Due to this differential sedimentation pattern or behaviour when agglutinated vs. when non-agglutinated, the particles of the first aspect may be used as a potential alternative of RBC in hemagglutination assays. Thus, the particles described herein act as synthetic erythrocytes, also known as an erythrocyte-mimicking reagent, and allow overcoming current drawbacks associated to this technology and mentioned in the background. Therefore, the invention also relates to the use of the particles of the first aspect as means or reagents in hemagglutination-like assays in any of their variations.

Thus, in a second aspect the invention relates to a method for detecting and/or characterizing pathogens (or pathogen antigens) present in an isolated test sample, said method comprising:
(a) contacting in a vessel comprising walls and a bottom, and in a buffered aqueous media, the sample (or a sample dilution series) where pathogens (or pathogen antigen) are to be detected, with a coloured solid particle as defined above in the first aspect;
(b) allowing the particles to sediment for a time from 1 to 20 minutes after the contacting step;
(c) determining the sedimentation pattern of the particles, wherein particle sedimentation on the bottom or walls of the vessel indicates lack of pathogen in that sample (or presence of pathogen at concentrations lower than the assay limit of detection), while the lack or absence of sedimentation is indicative of agglutinated particles, which means that interaction between the pathogen ligand on the surface of the particle and the pathogen (or pathogen antigen) in the sample has taken place because of an specific recognition between the pathogen (or pathogen antigen) and the ligand.

With this method, indeed, only 20 min, or even 10 min are needed to determine which sedimentation pattern of the particles has taken place. Indeed, the steps (a) contacting and (b) allowing to sediment take place in 20 min, even in 10 minutes maximum. The method includes, therefore, the contacting or mixture of the particles with the sample and the allowance of sedimentation step to occur for a period from 1 to 20 min, in particular from 1 to 10 min, prior to the determination of the sedimentation pattern.

In a third aspect, the invention relates to a method for detecting and/or characterizing pathogen-binding antibodies present in an isolated sample, said method comprising:
(a) contacting in a vessel comprising walls and a bottom, and in a buffered aqueous media, the sample where antibodies are to be detected, or a sample dilution series, with pathogen or pathogen antigens, and with a coloured particle as defined in the first aspect;
(b) allowing the particles to sediment for a time from 1 to 20 minutes after the contacting step;
(c) determining the sedimentation pattern of the particles, wherein particle sedimentation on the bottom or walls of the vessel indicates presence of pathogen-binding antibodies in that sample, which inhibited pathogen (or pathogen antigen) binding to the particles and particle agglutination; while the lack of sedimentation is indicative of particle agglutination by pathogen (or pathogen antigen) binding, and thus absence of pathogen-binding antibodies in the sample (or presence of pathogen-binding antibody at concentrations lower than the assay limit of detection).

As previously indicated, steps (a) and (b) take place in 20 minutes, even in10 minutes maximum, and then sedimentation pattern is visualized.

Finally, another aspect of the invention is the use of a kit for detecting and antigenically characterizing pathogens present in an isolated test sample, said kit comprising the coloured particles as defined in the first aspect, one or more vessels with a V-shaped or U-shape bottom, optionally a battery of pathogen antigen standards, optionally a battery of antibody of antiserum standards, and optionally instructions to carry out the method as defined above.

### Brief Description of Drawings

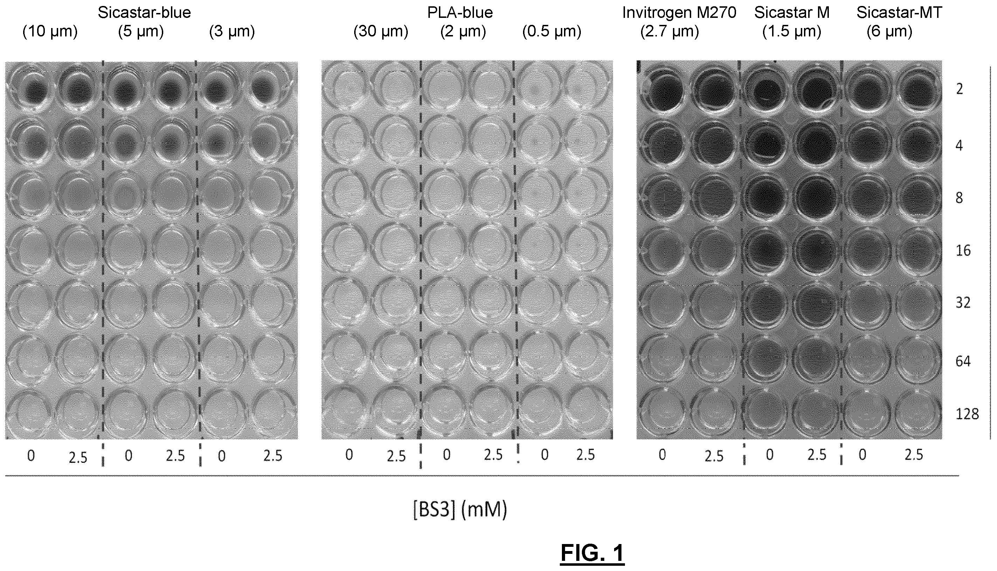

FIG. 1, related to Example 1, shows pictures with examples of the sedimentation patterns of 9 types of commercially available beads (nano- and microparticles) displaying amino or carboxyl groups on surface of the beads (Table 1). Beads were serially diluted 1:2 with PBS, in the absence or in the presence of a chemical agglutinating agent (BS3 at 0 and 2.5 mM concentrations, respectively; or EDC plus NH₂-PEG-NH₂ at 0 and 0.5 mg/ml each, respectively), and were allowed to settle down in U-shaped wells.

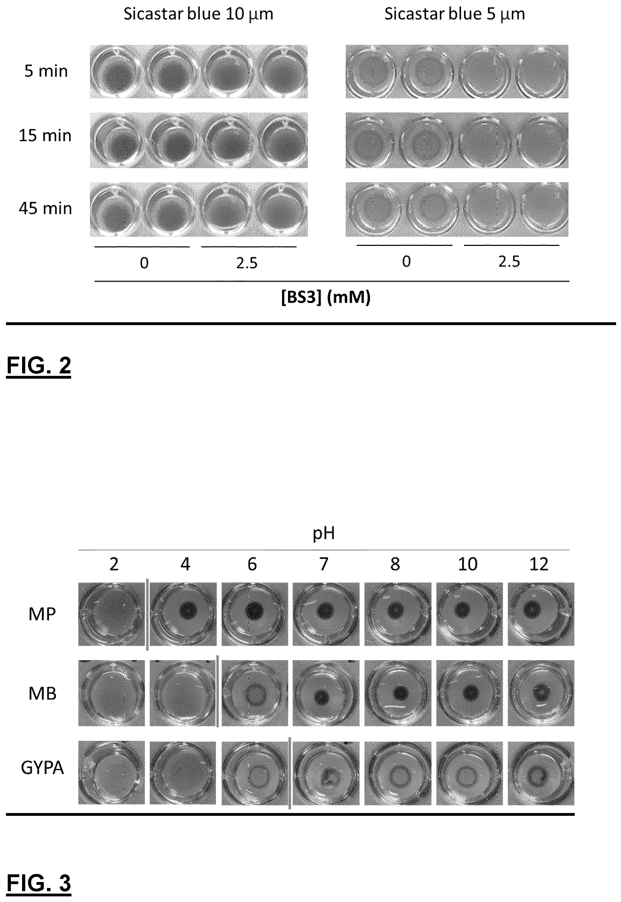

FIG. 2, related to Example 1, shows pictures of the beads sedimentation pattern in the presence and in the absence of an agglutinating agent after allowing them to settle down for 5-45 min.
FIG. 3, related to Example 3, shows the sedimentation pattern displayed by Sicastar blue beads (10 µm in diameter, ø), modified with different sialylated proteins and allowed to settle down while suspended in buffers of different pH. Isoelectric point from the sialylated proteins is marked with a red bar.

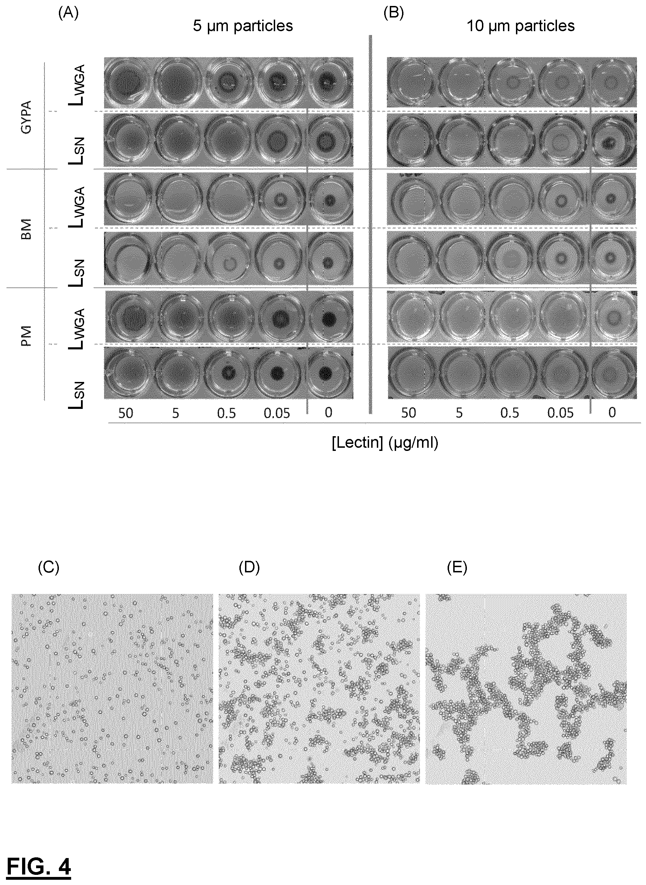

FIG. 4, related with Example 4, shows changes in sedimentation pattern of sialylated beads (displaying on surface PM, BM or GYPA) in the presence of a 10-fold serial dilution of two types of lectin, WGA and SNA. A fixed amount of sialylated beads was added per plate well (840 µg of 5 µm particles (A; equivalent to approximately 7.35 E6 particles), or 750 µg of 10 µm (B; equivalent to approximately 8.25 E5 particles)) and U-shaped wells were used. Microscopic images of BM-conjugated particles incubated in PBS (C), 1 µg/mL of WGA lectin (D) and in 10 µg/mL of WGA lectin (E).

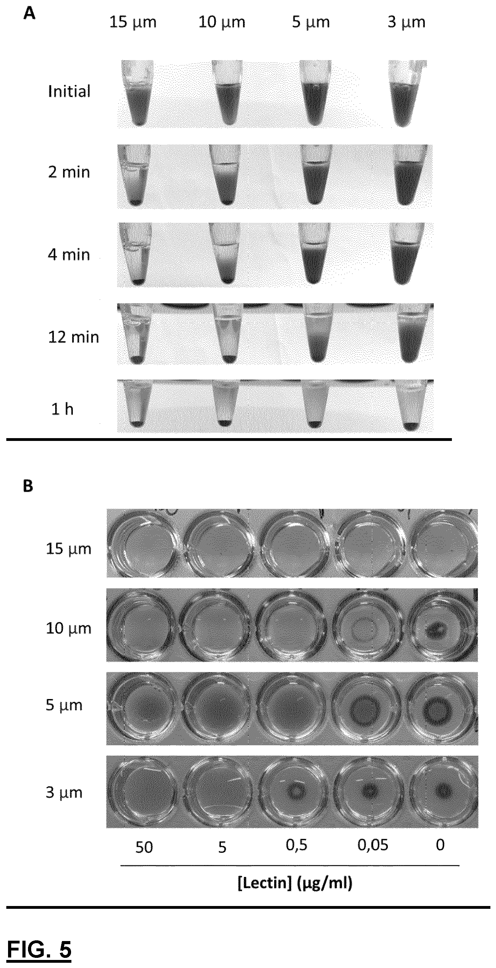

FIG. 5, related with Example 5, shows the effect of bead size in sedimentation.

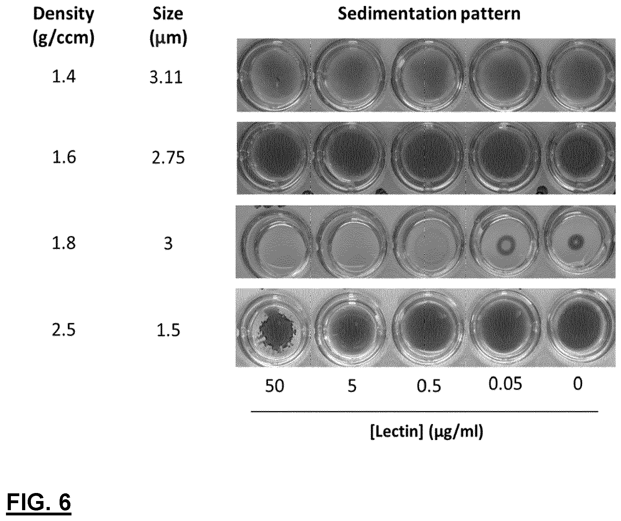

FIG. 6, related to Example 6, summarizes the effect of bead density in sedimentation.

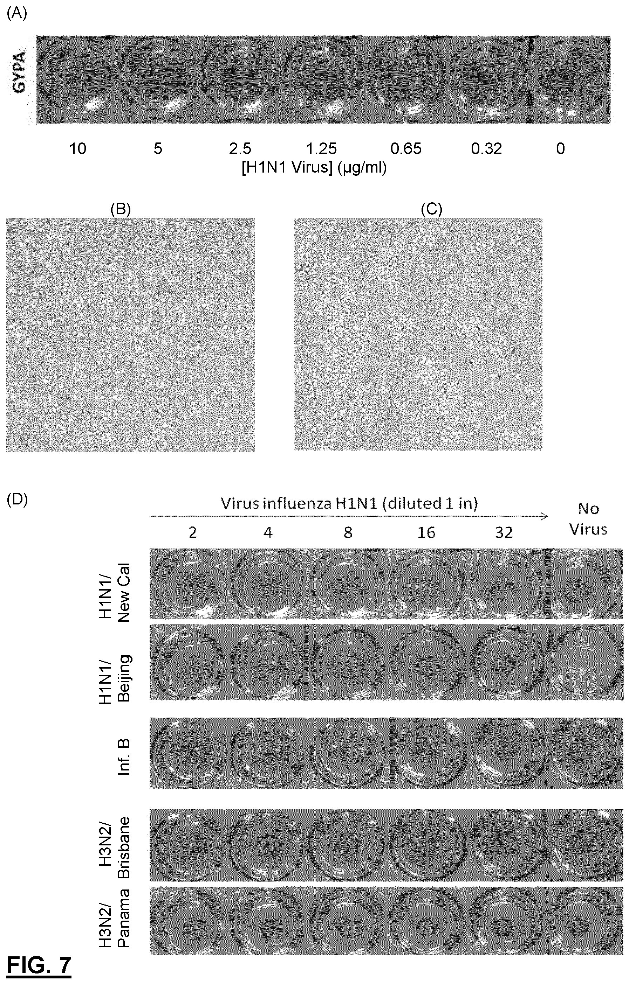

FIG. 7, related with Example 7, shows changes in the sedimentation pattern of sialylated beads generated by the presence of influenza A(H1N1) virus. (A) Sedimentation of GYPA-conjugated particles in U-shaped wells in the absence and in the presence of increasing concentrations of influenza A(H1N1) virus. (B-C) Microscopic images of GYPA-particles agglutinated in the presence of influenza (C), compared to non-agglutinaded or non-net-forming beads in the absence of the virus (B). (D) The behaviour of GYPA-particles was studied further in the presence of four more viral strains (A/Beijing/262/95 (H1N1), A/Panama/2007/99 (H3N2), A/Brisbane/10/07 (H3N2) and influenza B/Victoria/504/00).

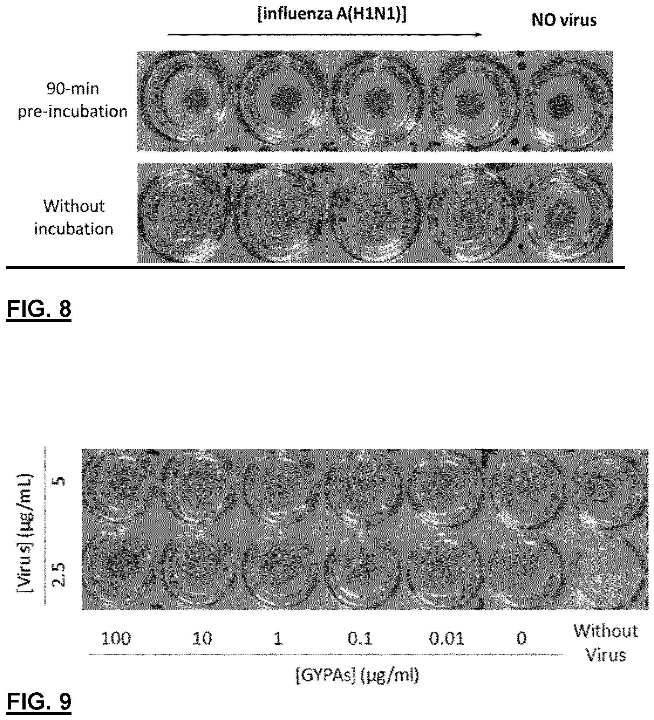

FIG. 8, related to Example 7, shows that carrying a pre-incubation of the GYPA-particles with the virus did not improve assay performance.
FIG.9, related with Example 7, shows an image of a competitive assay with soluble GYPA added to the medium. GYPA beads were incubated with a fixed concentration of influenza A(H1N1) virus (2.5 or 5 µg/mL) and increasing concentrations of GYPA in solution. Virus-induced agglutination was inhibited in the presence of high concentrations of soluble GYPA. The concentration of GYPA needed to inhibit agglutination was proportional to the concentration of virus.

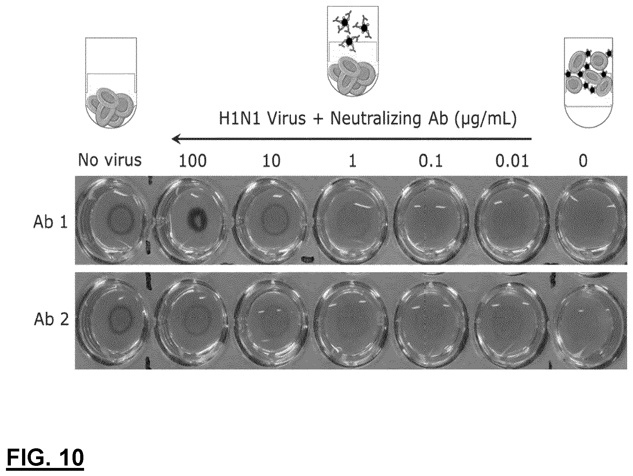

FIG. 10, related with Example 8, shows an agglutination inhibition assay in the presence of two neutralizing antibodies (Ab1 and Ab2) and with GYPA-particles.

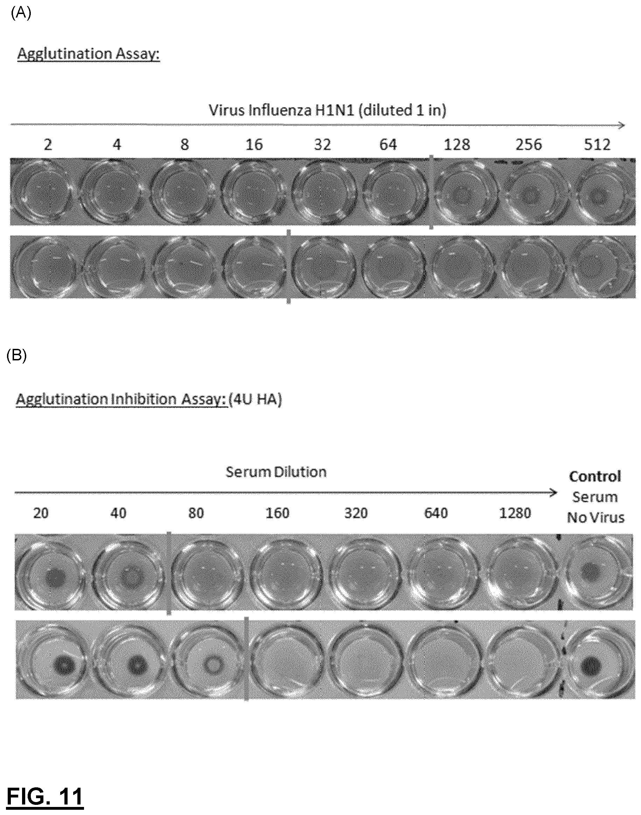

FIG. 11, related with Example 9, displays the results obtained in parallel when testing a fixed amount of either human erythrocytes or GYPA-particles in the presence of increasing concentrations of influenza A/New Caledonia/20/99 (H1N1) virus (A). An agglutination inhibition assay performed in parallel with human erythrocytes and GYPA-particles displayed comparable results (B).

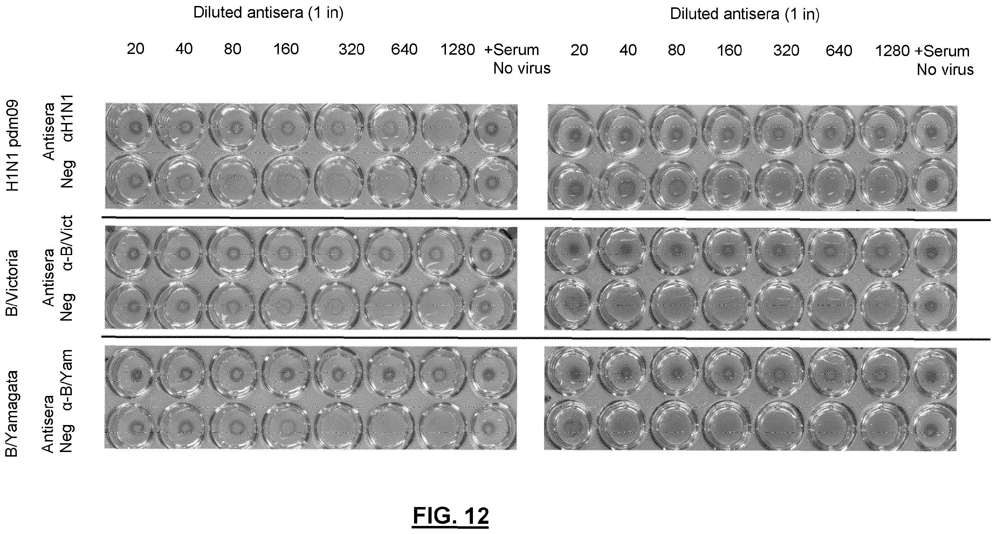

FIG. 12, related to Example 10, displays the results of a series of agglutination inhibition assays performed in parallel with erythrocytes-mimicking beads, called also synthrocytes in this description (left, 5 min), or human RBCs (right, 60 min), using reference virus and antisera (previously treated with RDE) provided by the WHO, though the CDC, Atlanta.

### Detailed description of the invention

Unless otherwise stated, all terms as used in this application shall be understood in their ordinary meaning as known in the art. Other more specific definitions for certain terms as used in the present application are as set forth below and are intended to apply uniformly throughout the specification and claims unless an otherwise expressly set out definition provides a broader meaning.

As used herein, the indefinite articles "a" and "an" are synonymous with "at least one" or "one or more." Unless indicated otherwise, definite articles used herein, such as "the," also include the plural of the noun.

The term "sedimentation" relates to the tendency for particles in suspension to settle out of the fluid in which they are suspended and come to rest against a barrier, for example the bottom/base of a vessel, or against a cluster or grouping (enucleation) of particles. This is due to their motion through the fluid in response to the forces acting on them: these forces can be due to gravity, centrifugal acceleration, electromagnetism, or specific recognition of the particles for compounds in certain surfaces. Sedimentation of a particle can vary depending on the media wherein it is in suspension and also depending on the interactions that are stablished with the components of the media. Therefore, different patterns of sedimentation (sedimentation patterns or sedimentation behaviour) can be observed for particles of similar features (diameter, surface, material...) that are embedded or suspended in media of different composition.

The expression "determining the sedimentation pattern of the particles" means to see if agglutination (no sedimentation on the bottom or walls of a vessel) takes place or if, on the contrary, sedimentation of particles occurs on the bottom/walls of the vessel.

"Agglutination of the particles" relates to the formation of a net of interacting particles and pathogens (or pathogen antigen), which is visualized as a turbulence in the buffered media or in the isolated sample where particles are added. According to this description, the term "agglutination" is defined as including also the interaction of any substance in a buffered media with one or more of the particles. This interaction takes place due to different physical phenomena, including ionic forces or electrostatic interactions, van der Waals forces, and in some case covalent bindings. When several particles interact with one or more substances (i.e. pathogens) in the buffered media, this interaction is easily visualized (turbulence) and is generically called "agglutination" taking the word of the tests carried out with RBC.

The term "material mass density" relates to the mass per volume unit of the particles (mainly of the core and surface core of the particles) and it is indicated in grams per cubic centimetre (g/cc, g/ccm or g/cm³). It is measured according to standard methods. In particular, density can be measured by centrifuging the particles in a density or viscosity gradient of a material (i.e a cesium chloride column), said gradient increasing from top of the centrifuge tube to bottom (i.e. higher densities/viscosities of the material are on the bottom of the centrifuge tube). Particle density is that of the material where the particle is retained after centrifugation.

"Particles" described herein are microparticles, which means that at least two dimensions are at the microscopic scale, particularly with all three dimensions at the microscopic scale. As regards the shape of the microparticles described herein, there are included spheres, polyhedral and rod-shape. Particularly, when the microparticle is substantially rod-shaped with a substantially circular cross-section, such as a microwire or microtube, the "microparticle" refers to a particle with at least two dimensions at the microscale, these two dimensions being the cross-section of the microparticle. In a particular embodiment, the particles are spherical. As used herein, the term "size" of the particle or diameter refers to a characteristic physical dimension. For example, in the case of a microparticle that is substantially spherical, the size of the microparticle corresponds to the diameter of the microparticle. When referring to a set of microparticles as being of a particular size, it is contemplated that the set can have a distribution of sizes around the specified size. Thus, as used herein, a size of a set of microparticles can refer to a mode of a distribution of sizes, such as a peak size of the distribution of sizes. In addition, when not perfectly spherical, the diameter is the equivalent diameter of the spherical body including the object. This diameter is generally referred as the "hydrodynamic diameter", which measurements can be performed using a Wyatt Möbius coupled with an Atlas cell pressurization system. Transmission Electron Microscopy (TEM) images do also give information regarding diameters.

For "coloured" is to be understood that the particles have a colour within the white and all the visible light spectrum. "Coloured" means that particles are visible by the naked eye, without requiring any external equipment, such as lasers, fluorescence readers, or detectors of any type for visualization. Thus, the particles are of visible colour by the naked human eye. Particular coloured particles are blue and red particles that can easily be contrasted in aqueous buffered media where the particles of the invention are usually employed, and form well defined sediments that are easy to study by the naked eye without any external visualization equipment. In particular, they are non-fluorescent but of a colour visible by naked human eye.

The terms "pathogen", "pathogen antigen" and "antigen" are used interchangeably in this description. Although in the oldest and broadest sense, a pathogen is anything that can produce disease, in this description the term encompasses also antigens (which are otherwise only a portion of the pathogen components) which non-necessarily are from any source causing disease. It also encompasses those well-characterized particular antigens of pathogens or organisms causing diseases.

"Antigen" or "pathogen antigens", used interchangeably in this description, relate to structures, usually of protein nature which can be modified with sugar moieties, specifically bound by antibodies (Ab) or a cell surface version of Ab ~ B cell antigen receptor (BCR), or by a cell surface compound that is used by pathogens to contact such cells. An epitope, also known as antigenic determinant, is the part of an antigen that is recognized by the immune system, specifically by antibodies, B cells, or T cells, or that pathogens use to target the cells. When the expression "pathogen antigens" is used in this description, it particularly relates to isolated antigens of the pathogens, thus to the particular protein or fragment thereof that is known to interact with cells in the organisms. Different pathogen constituents can behave as antigens, including structural and non-structural components, such as enzymes, toxins, metabolites, and so on.

The term "pathogen ligand" or "antigen ligand", are used interchangeably in this description, and they relate to particular structures immobilized on the surface of the particle core, and which have the capability of interacting with molecules, generically of protein nature or modified proteins (e.g. glycosylated proteins). This interaction between antigen/pathogen ligand and the molecules takes place in the media (i.e. aqueous buffered media, isolated biological samples or isolated environmental samples) where the particles are added.

The term "pathogen antigen standards" includes series of known, tittered and well-characterized antigens of pathogens that are employed to see if a particular isolated test sample contains any element (cells, antibodies, particles, receptors) capable of interacting with any of them.

Any of the expressions "detecting and/or characterizing pathogens" or "detecting and/or characterizing pathogen-binding antibodies", relates to the action of being capable of indicating that an antigen or an antibody is effectively present in a sample (detecting) and/or to the capability of further obtaining particular information of the type of pathogen or pathogen-binding antibody (i.e.: antigenic determinants of the pathogen or particular antibodies recognizing a particular antigenic determinant of a pathogen).

For a "buffered solution", "buffered media" or "buffered aqueous media", used interchangeably, is to be understood any liquid comprising water and the appropriate pH buffering compounds to fix a pH range in said liquid. pH adjusting agents include citrate salts (citric acid/citrate buffer), phosphate salts (phosphoric acid/phosphate buffer), borate salts (boric acid/borate buffer), Tris (Tris(hydroxymethyl)aminomethane), HEPES (4-(2-hydroxyethyl)-1-piperazineethanesulfonic acid), HEPBS (N-(2-Hydroxyethyl)piperazine-N'-(4-butanesulfonic acid)), MES (2-(N-morpholino)ethanesulfonic acid), MOPS (3-(N-morpholino)propanesulfonic acid), CHES (N-Cyclohexyl-2-aminoethanesulfonic acid), PIPES (Piperazine-N,N'-bis(2-ethanesulfonic acid)), and mixtures thereof. pH buffers may in addition comprise amino acids, in particular arginine, lysine, glycine and an amine-derived compound selected from methylglucamine and trometamol, and mixtures thereof. Particular buffers for media including or involving viral particles are phosphate-buffered saline (PBS), optionally supplemented with amino acids, proteins, diluted serum, and so on.

Throughout the description and claims, the term "-(C₁-C₈)-alkyl radical", shall be construed as linear or branched. It includes, any of the radicals methyl, ethyl, n-propyl, iso-propyl, cyclopropyl, n-butyl, sec-butyl (or 1-methylpropyl), isobutyl (or 2-methylpropyl), tert-butyl (or 1, 1-dimethylethyl), n-pentyl, tert-pentyl (or 2-methylbutan-2-yl), neopentyl (or 2,2-dimethylpropyl), isopentyl (or 3-methylbutyl), sec-pentyl (or pentan-2-yl), 3-pentyl (or pental-3-yl), n-hexyl, isohexyl (or 4-methylpentyl), tert-hexyl, sec-hexyl, n-heptyl, tert-heptyl, isoheptyl, sec-heptyl, n-octyl, tert-octyl, sec-octyl, and isooctyl. In the same way, the term "-(C₁-C₈)-alkoxyl" shall be construed as linear or branched and it includes methoxyl, ethoxyl, n-propyloxy, iso-propyloxy, cyclopropyloxy, n-butyloxy, sec-butyloxy (or 1-methylpropyloxy), isobutyloxy (or 2-methylpropyloxy), tert-butyloxy (or 1,1-dimethylethyloxy), n-pentyloxy, tert-pentyloxy (or 2-methylbutan-2-oxyl), neopentyloxy (or 2,2-dimethylpropyloxy), isopentyloxy (or 3-methylbutyloxy), sec-pentyloxy (or pentan-2-oxyl), 3-pentyloxy (or pental-3-oxyl), n-hexyloxy, isohexyloxy (or 4-methylpentyloxy), tert-hexyloxy, sec-hexyloxy, n-heptyloxy, tert-heptyloxy, isoheptyloxy, sec-heptyloxy, n-octyloxy, tert-octyloxy, sec-octyloxy, and isooctyloxy.

As above indicated, a first aspect described herein is a coloured particle comprising:
- a core with a surface, said core of a material with a mass density from 1.5 to 2.5 g/cc; and a diameter from 3 to 10 µm, and wherein at least the surface is coloured and it comprises one or more functional groups selected from -COO-R¹, -NR² R³ , and combinations thereof, wherein R¹ is independently selected from H, and (C₁-C₈)-alkyl, and R²and R³ are independently selected from H, (C₁-C₈)-alkyl, and (C₁-C₈)-alkoxyl; and
- a pathogen ligand immobilized on the surface of the core.

In a particular embodiment, the particles are a solid filled particle, which means that the core is not hollow although it can be porous. Thus, in another particular embodiment, the particles are solid porous particles of one or more materials in combination. These particles can be supplied in suspension in appropriated buffered systems, including polar solvents (aqueous buffered media) and organic solvents. In particular, the particles are of an homogeneous single material in their whole volume, which means that the external surface an inner core are of the same material.

In a particular embodiment, the particles comprise a spherical or semi-spherical core. This means that particles are spherical. In another particular embodiment, the diameter of the core of particles is selected from 3.0, 3.5, 4.0, 4.5, 5.0, 5.5, 6.0, 6.5, 7.0 7.5, 8.0, 8.5, 9.0, 9.5 and 10.0 µm. In another particular embodiment, the diameter of the core of particles is from 3 to 10 µm. More in particular, it is from 5 to 10 µm. Diameters indicated in this description correspond to actual diameters measured by electron microscopy before any immobilization of a pathogen ligand. Particle diameter is thus defined by the core diameter and the immobilized pathogen ligand, although in general pathogen ligands (of nanometric scale) account only slightly to the micrometric size of the particle core. Other ways to measure particle diameter include dynamic light scattering (DLS) that gives the so-called hydrodynamic diameter and that corresponds to the size in a particular media (co-solvation of particles with media).

In another particular embodiment, optionally in combination with any embodiment above or below, the material mass density of the particle core is selected from 1.5, 1.6, 1.7, 1.8, 1.9, 2.0, 2.1, 2.2, 2.3, 2.4 and 2.5 g/cc. In another particular embodiment, optionally in combination with any embodiment above or below, the material mass density of the particle core is from 1.5 to 2.0 g/cc. In another particular embodiment is from 1.8 to 2.0 g/cc. More in particular, the material mass density of the particle core is of 1.8 g/cc.

As will be illustrated in the examples below, the particular combination of mass density of the particle with the diameter of the particle comprising the immobilized pathogen ligand do mimic RBC behaviour in agglutination assays in particular buffered media. Advantageously, the particles described herein sediment quickly, which allows a fast test of samples including pathogens (or pathogen antigen) or antibodies recognizing said pathogens. Desired sedimentation times for visualizing if interaction of the particles with particular components of the media wherein they are suspended take place, include 1 min and up to 10 min. 20 minutes also works, although visible sedimentation pattern is clearly visible in less minutes. In contrast, the usual sedimentation times required for RBC are from 30 min (avian RBC) to 60 min (mammalian RBC).

In a more particular embodiment, the core has a material mass density from 1.5 to 2.0 g/cc; and a diameter from 3 to 10 µm.

As indicated in the summary and due to immobilized pathogen ligand, and also to the one or more functional groups on the surface of particle core, particles have a particular surface charge in a buffered aqueous media and depending on the pH of the media. Particles are, in particular embodiments, conceived to have a net surface charge (positive or negative) in particular pH conditions. In a particular embodiment, particles are conceived to have a negative surface charge when placed in buffered aqueous media with a pH higher than the net isoelectric point (Ip) of the particle surface constituents (i.e. ligand and functional groups).

Although several materials can be used, in a particular embodiment the core of the particle described herein and the core surface are made of a material selected from the group consisting of glass, silica, sand, wood, fiber, natural polymer, synthetic polymer, and combinations thereof. In a more particular embodiment, the particle is made of silica.

In a particular embodiment, R¹ and R² and R³ are H. In another particular embodiment, the surface of the core of the particle comprises as functional chemical groups aldehyde groups, amine groups, and thiol groups.

In yet another particular embodiment, optionally in combination with any embodiment above or below, the pathogen ligand is a compound comprising pathogen-recognizing molecular patterns. A pathogen-recognizing molecular pattern is any compound or group of compounds usually involved in the recognition of cells or pathogens, or antigens in said pathogens. Particular pathogen-recognizing molecular patterns are selected from oligosaccharides (2-10 monosaccharide units), aptamers, acids (such as sialic acids, oleanolic acid, and so on), oligonucleotides (2-30 nucleotides), synthetic antibodies, phage-displayed peptides, monosaccharides, peptides (from 2 to 30 amino acids), proteins (at least 31 amino acids), and glycosylated amino acids, peptides or proteins. Particular monosaccharides are sialic acid. Pathogen ligands are thus, in a particular embodiment, molecules that comprise sialic acid or glycoproteins comprising sialic acid. Molecules comprising sialic acid or other monosaccharides, other than glycoproteins comprising sialic acid, and also involved in recognition of antigens in pathogens are, in a particular embodiment, polymeric compounds functionalized with the sugar moieties disposed defining such a conformational assembly that mimics glycosylated amino acids (i.e.: sialylated amino acids) of glycoproteins.

In a particular embodiment, the pathogen ligand is sialic acid.

In another particular embodiment, the pathogen ligand is a protein. In another particular embodiment is a glycoprotein. More in particular it is a sialylated protein.

Sialylated proteins are recognized by several viral receptors and/or virus molecules. It is widely known that the glycoprotein hemagglutinin (HA) of viral envelopes, and in particular that of influenza virus, binds to cellular receptors comprising sialic acid in the membrane of erythrocytes in hemagglutination assays. In a more particular embodiment, the sialylated protein immobilized on the particle surface is a sialylglycoprotein selected from glycophorin, podocalyxin, mucin, transferrin, fetuin, gangliosides, luteinizing hormone (LH), follicle-stimulating hormone (FSH), and human chorionic gonadotropin (hCG). These are particular examples, but as a general rule any sialylated protein will work.

In another particular embodiment, the sialylated protein is able to recognize viral receptors (such as proteins in their envelope) selected from a virus of the group consisting of influenza A virus, influenza B virus, influenza C virus, coronavirus, measles virus, Newcastle Disease virus, hepatitis virus, rhinoviruses, adenoviruses, human parainfluenza viruses, human respiratory syncytial virus, enteroviruses, metapneumovirus, reovirus, rotavirus, bluetongue virus.

In another particular embodiment, the sialylated protein is able to recognize viral receptors (such as proteins in their envelope) selected from a virus of the group consisting of influenza A virus, influenza B virus, influenza C virus, human coronavirus, bovine coronavirus, measles virus, Newcastle Disease virus, mouse hepatitis virus, equine rhinitis A, reovirus, rotavirus, bluetongue virus. In a particular embodiment the virus detected is influenza virus.

Particular silica particles with sialylglycoproteins immobilized on their surface have been tested against several strains of influenza virus with good results, in terms of being useful in hemagglutination-like assays or HAl-like assays. Thus, in another particular embodiment of the first aspect, the particle is made of a silica core, more in particular a porous coloured silica, and it comprises immobilized on its surface glycophorin. In yet a more particular embodiment, glycophorin is glycophorin A.

With regard to immobilization of the pathogen ligand, the operator skilled in the art will know different methods. In a particular embodiment of the particle of the first aspect, immobilization is carried out by means of chemical groups (functional groups) on the surface of the particle, said chemical groups allowing forming chemical covalent bonds with those amino acids of the sequence of the ligand including reactive groups, such as carboxylic and/or amine groups in the side chain of alpha carbon.

In this particular embodiment where chemical covalent bonds are established between the pathogen ligand and the particle surface, above-mentioned chemical groups on surface of the core are employed. They are selected from -COO-R¹, -NR² R³ and combinations thereof, wherein R¹ is independently selected from H, and (C₁-C₈)-alkyl, and R² and R³ are independently selected from H, (C₁-C₈)-alkyl, and (C₁-C₈)-alkoxyl. In a particular embodiment R¹ and R² and R³ are H.

Carboxylated groups (-COO-R¹ with R¹ as H) on the particle surface are easily reacted with the known tandem of molecules 1-ethyl-3-(-3- dimethylaminopropyl) carbodiimide hydrochloride (EDC) and N-hydroxysuccinimide (NHS) for the immobilisation of large biomolecules. Amine-bearing particles (amine groups on the particle surface) are reacted with glutaraldehyde for biomolecule immobilization.

Alternatively, the ligand can be indirectly incorporated on the particles, for instance by using particles modified with biotin-binding reagents (avidin, streptavidin, neutravidin, captavidin, anti-biotin antibodies, etc), anti-FITC antibodies, or anti-digoxin antibodies, to cite some examples, to incorporate on surface a ligand that had been previously modified with biotin, FITC, digoxigenin, etc.

If convenient, the particle comprises a linker molecule between said particle surface with the chemical groups (-COO-R¹ and/or -NR² R³) and the pathogen ligand. Examples of linker molecules include those known in prior art, and widely used to optimize immobilization and for providing ligand disposition made more accessible and non-sterically compromised.

As above indicated, due to this differential behaviour between non-agglutination and agglutination (lack of sedimentation on walls/bottom of a vessel), the particle of the first aspect may be used to substitute animal RBC in hemagglutination assays. Thus, the particles as defined in the first aspect are used as erythrocyte-mimicking reagents. In particular, they are used as means or reagents in for hemagglutination-like assays, even more in particular these assays selected from an hemagglutination (HA) assay and a hemagglutination inhibition (HAI) assay.

Thus, the second aspect of the invention relates to a method for detecting and/or characterizing pathogens present in an isolated test sample, said method comprising:
(a) contacting in a vessel comprising a wall and a bottom, and in a buffered aqueous media, the sample where pathogens are to be detected, with a coloured solid particle as defined above in the first aspect;
(b) allowing the particles to sediment for a time from 1 to 20 min, in particular from 1 to 10 min after the contacting step, more in particular from 1 to 5 min;
(c) determining the sedimentation pattern of the particles, wherein particle sedimentation on the bottom or walls of the vessel indicates lack of pathogen in that sample, or presence of pathogen at concentrations lower than the assay limit of detection, while the lack or absence of sedimentation on walls and /or bottom of the vessel is indicative of agglutinated particles, which means that interaction between the pathogen ligand on the surface of the particle and the pathogen in the sample has taken place and the pathogen has been recognized by the pathogen ligand.

In a particular embodiment, step (a) is carried out with dilution series of the sample, and the method further comprises step (d) of determining pathogen agglutination titter from the sample dilutions in which particles displayed a change in the sedimentation pattern (agglutination vs sedimentation on the bottom/wall of the vessel).

In another particular embodiment of this second aspect, in step (a) a competitive ligand for the pathogen is added, competing for the interaction of the pathogen ligand on the surface of the particle with the pathogen in the sample.

In this particular embodiment, the method is a method for antigenically characterizing pathogens and it comprises the steps of:
(a) contacting in a vessel comprising a wall and a bottom, in a buffered aqueous media, a coloured solid particle as defined above in the first aspect with a first sample comprising pathogens (or pathogen antigen) and a second sample comprising competitive pathogen-binding ligands with null cross-reactivity with the particles;
(b) allowing the particle to sediment within a time from 1 to 20 min, more in particular from 1 to10 min, and even more in particular from 1 to 5 min; and
(c) determining the sedimentation pattern of the particle, wherein if sedimentation pattern shows agglutinated particles, this means that the pathogen (or pathogen antigen) has been recognized by pathogen ligand on particle surface through interaction between the said immobilized pathogen ligand and the pathogen (or pathogen antigen) in the first sample, and if sedimentation pattern shows non-agglutinated particles, thus sedimentation on the bottom of the vessel, this means that competitive ligand of second sample specifically recognized one or more antigens of the pathogen of the first sample through interaction of said competitive ligand of second sample and the pathogen of the first sample.

"Null cross-reactivity" relates to the fact that the competitive pathogen-binding ligand does not interact with the particles, thus, does not induce nonspecific agglutination.

In a more particular embodiment of the method for detecting, and optionally antigenically characterizing a pathogen in a test sample, said sample is selected from animal or plant origin, or it is an environmental sample. Particular examples of samples of animal or plant origin are selected from animal respiratory tract, serum, plasma, blood cells, samples from avian farms (faeces, blood, meal), animal cell cultures, vaccines, blood derivatives (platelet rich plasma, platelet poor plasma, leukocyte rich or poor plasma, etc.). Animal includes human according to this description. Environmental samples include, in particular, water (wastewater, common water), and material from refrigeration or cooling towers. "Test sample" is a questioned isolated sample in which certain parameters want to be determined (i.e., presence of pathogens (or pathogen antigen), presence of antibodies, etc.).

In yet a more particular embodiment, the sample is an isolated test sample from a subject, in particular from a mammal, more in particular a human. In this particular embodiment therefore, the method is an in vitro method for detecting and optionally antigenically characterizing the current or previous presence of a pathogen in a subject.

In the particular case in which the method is carried out with a first sample comprising pathogens (or pathogen antigen) and a second sample comprising competitive ligands of the pathogen, said first and second samples are independently selected from isolated test samples of a subject.

In a more particular embodiment, the first sample is a sample isolated from a subject and the second sample is a buffered solution comprising a competitive ligand, which specificity for certain antigens of a pathogen is known. Thus, with this embodiment, correct antigenic characterization of the pathogen (not only its detection) is accomplished. The operator skilled in the art will understand that this method is carried out with one or more competitive ligands with differing recognition specificity (recognizing different antigens) for the pathogen, and so the method allows an accurate identification of the pathogen in the test sample of the subject.

On the alternative, also in the particular case in which the method is carried out with a first sample comprising pathogens (or pathogen antigen) and a second sample comprising competitive pathogen-binding ligands, the first sample is a buffered solution comprising one or more pathogens (or pathogen antigens), and the second sample is a sample isolated from a subject. With this variation of the method, the specific immune response of a subject to certain pathogens can be detected. Indeed, interaction of the pathogen of the first sample and the particles will be avoided if the sample of the subject comprises competitive ligands for this pathogen (i.e., specific antibodies for the pathogens of the first sample).

In a more particular embodiment, particular isolated test samples of subjects are selected from tissue biopsies containing cells (i.e. cells secreting antibodies and or cells infected with the pathogen), a biofluid sample, in particular selected from plasma, serum, whole blood, saliva, semen, respiratory specimen, cerebral spinal fluid (CSF), tears, mucus, sweat and milk. More particular biofluid samples are selected from plasma, serum, and whole blood.

In a more particular embodiment of the methods of the second aspect, optionally in combination with any embodiment above or below, the competitive ligand of the pathogen is selected from an antibody specifically recognizing one or more antigens of the pathogen, a free pathogen ligand, a synthetic ligand (such as oligosaccharides, sialic acids, aptamers, oligonucleotides, synthetic antibodies, peptides, phage-displayed peptide) and combinations thereof.

In yet a more particular embodiment, the competitive ligand is an antibody that specifically recognizes one or more antigens of the pathogen. These antibodies are selected from monoclonal antibodies, polyclonal antibodies or fragments of any of these polyclonal or monoclonal antibodies specifically recognizing one or more antigens of a particular pathogen. For a fragment of an antibody, being from a polyclonal or from a monoclonal antibody, is to be understood any of the F(ab), F(ab') and Fv fragments.

For "antibody" (abbreviated Ab), also known as an immunoglobulin (Ig), it is to be understood as a large, Y-shaped protein produced mainly by plasma cells that is used by the immune system to neutralize pathogens such as bacteria and viruses. The antibody specifically recognizes a unique molecule of the pathogen, called an antigen, via the Fab's variable region. Each tip of the "Y" of an antibody contains a paratope (analogous to a lock) that is specific for one particular epitope (similarly analogous to a key) on an antigen, allowing these two structures to bind together with precision. The expression "battery of antibody or antiserum standards" relates to series of antibodies that are well-characterized in terms of sensitivity and specificity (cross-reactivity) and that are used to determine if a test sample contains any element (cell, pathogen) capable of interacting (to be recognized) by them. These batteries are composed of the serum of animals immunized with specific pathogens/antigens in a particular embodiment. These batteries are composed of commercially available antibodies in another particular embodiment.

Depending on said specific recognition, the method of the invention even allows discrimination between serotypes among pathogens including several variations.

As indicated, another aspect of the invention is a method for detecting and/or characterizing pathogen-binding antibodies present in an isolated sample, said method comprising:
(a) contacting in a vessel comprising a wall and a bottom, and in a buffered aqueous media, the sample where antibodies are to be detected, with pathogen or pathogen antigens, and with a coloured particle as defined in the first aspect;
(b) allowing the particles to sediment for a time from 1 to 20 min, in particular from 1 to10 min, and even more in particular from 1 to 5 min after the contacting step;
(c) determining the sedimentation pattern of the particles, wherein particle sedimentation on the bottom or walls of the vessel indicates presence of pathogen-binding antibodies in that sample, which inhibited pathogen binding to the particles and particle agglutination; while the lack of sedimentation on the bottom or walls of the vessel is indicative of particle agglutination by pathogen binding, and thus absence of pathogen-binding antibodies in the sample, or presence of antibodies at concentrations lower than the assay limit of detection.

In a particular embodiment of the method for detecting and/or characterizing pathogen-binding antibodies present in an isolated test sample, step (a) is carried out with dilution series of the sample, and the method further comprises a step (d) of determining antibody agglutination inhibition titter from the sample dilutions in which particles displayed a change in the sedimentation pattern (agglutination vs sedimentation on the bottom of the vessel).

In yet another particular embodiment of the method for detecting and/or characterizing pathogen-binding antibodies, step (a) is carried out with a battery of pathogens or pathogen antigens). This embodiment allows multiplexing the method, to detect particularly one or more of the antibodies in the isolated test sample (the sample where antibodies are to be detected).

In yet another particular embodiment of the method for detecting and/or characterizing pathogen-binding antibodies, step (a) is carried out in two phases by first a pre-incubation (contacting) of the sample with the antigens, and further adding the particles of the first aspect.

In another particular embodiment of the second and third aspects, step (a) is performed at a pH in which the particle has a net electrical surface charge, said charge provided by the combination of the isoelectric point (Ip) of pathogen ligand immobilized on the surface of the core of the particle and the charge of functional groups selected from -COOR¹, -NR²R³ on the particle surface.

The isoelectric point (Ip, pH(I), IEP), is the pH at which a particular molecule carries no net electrical charge or is electrically neutral in the statistical mean.

Particular pH for performing step (a) is that higher than the average isoelectric point of the particle, said average isoelectric point provided by the surface electrical charge of pathogen ligand immobilized on the surface of the core of the particle and the functional groups selected from -COOR¹, -NR²R³ also on the said core surface of the particle.

Any of the methods of the second and third aspects are, in particular, carried out at room temperature (18°C-26°C), although they could be carried out at a higher temperature as well (such as 37°C), and they are fast methods that are performed as indicated in from 1 to 10 minutes.

With the methods of the invention several pathogens (or pathogen antigen) can be detected in samples of several sources and the pathogens can be accurately and reliably characterized. In a particular embodiment of the method, the pathogen is selected from a bacteria, a virus, a protozoan, a fungus, a prion or a viroid.

In a more particular embodiment, the pathogen is a virus selected from the group consisting of influenza A virus, influenza B virus, influenza C virus, coronavirus, measles virus, Newcastle Disease virus, hepatitis virus, rhinoviruses, adenoviruses, human parainfluenza viruses, human respiratory syncytial virus, enteroviruses, metapneumovirus, reovirus, rotavirus, bluetongue virus.

In a more particular embodiment, the pathogen is a virus selected from the group consisting of influenza A virus, influenza B virus, influenza C virus, human coronavirus, bovine coronavirus, measles virus, Newcastle Disease virus, mouse hepatitis virus, equine rhinitis A, reovirus, rotavirus, bluetongue virus. Even in a yet more particular embodiment, the virus is selected from infuenza A virus, influenza B virus, influenza C virus and combinations thereof.

In even a more particular embodiment of the method, the pathogen is influenza virus and the coloured particle is a particle made of silica and comprises immobilized on its surface a sialylated protein as a pathogen ligand. In a more particular embodiment, the sialylated protein is a glycophorin, even more in particular is glycophorin A. In also another particular embodiment, the coloured particle comprises immobilized on its surface isolated or synthetic sialic acids. In also another more particular embodiment the silica particle has a material mass density of 1.8 g/cc and a hydrodynamic diameter of 10 µm.

Inventors realized that the methods of the invention dully operated in vessels whose vertical section corresponds to a plane in "V" or "U", thus that have a V- or U-shaped bottom. In a more particular embodiment, the vessel is a U-shaped bottomed vessel. These vessels allow good visual distinction of the sedimentation pattern being the particles agglutinated (not sedimented) or not agglutinated (sedimented on bottom/walls of vessel).

Coloured particles imply the advantage that they are easy detectable. This implies that no complex instrumentation is needed to carry out the methods of the invention. Nonetheless, agglutination or non-agglutination of the particles and their sedimentation patterns can also be monitored using particular instrumentation selected from spectrophotometers, allowing determining a differential UV-visible light absorption, light scattering or dispersion; electrochemical sensors (impedimetric, potentiometric, voltammetric, field-effect transistors), allowing determining changes in charge distribution upon antigen binding and particle agglutination and sedimentation; and quartz crystal microbalances and surface plasmon resonance (SPR) sensors, allowing determination of particle sedimentation on sensor surface. Advantageously, being the particles coloured, the method can be performed without any of these instrumentations.

Described herein are also adapted kits for carrying out the method of the second and third aspects and related embodiments, and another aspect is a kit for optionally detecting and antigenically characterizing pathogens present in an isolated sample, said kit comprising: the coloured particle as defined in the first aspect and related embodiments; one or more vessels with a V-shaped or U-shape bottom; and optionally instructions to carry out the method.

Forms also part of the invention the use of the particles of the first aspect in agglutination assays; and agglutination inhibition assays.

Throughout the description and claims, the word "comprise" and variations of the word are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Example 1. Selection of particles to produce an erythrocyte mimetic reagent (erythrocyte-mimicking reagent or synthrocyte)

### Materials:

The characteristics of the12 types of particles tested have been summarized in Table 1:

| | **Diameter (µm)** | **Surface** | **Particle** | **Material (color)** | **Density (g/ccm)** | **Provider** |
|---|---|---|---|---|---|---|
| **1** | 10 | NH₂ | Sicastar - blue | Silica (blue) | 1.8 | Micromod (Germany) |
| **2** | 5 | | | | | |
| **3** | 3 | | | | | |
| **4** | 5 | COOH | | | | |
| **5** | 30 | NH₂ | PLA-blue sicastar | Poly(D,L-lactic acid) (blue) | 1.0 | |
| **6** | 2 | | | | | |
| **7** | 0.5 | | | | | |
| **8** | 1.5 | NH₂ | Sicastar-M | Magnetic silica (brown) | 2.5 | |
| **9** | 6 | NH₂ | Sicastar-M-CT | Cluster-typed magnetic silica (brown) | 2.5 | |
| **10** | 2.75 | COOH | ProMag 3 | Polymer and iron oxide (brown) | 1.6 | Bangs Laboratori |
| **11** | 3.13 | COOH | ProMag HP 3 | Polymer and iron oxide (brown) | 1.4 | es Inc. (USA) |
| **12** | 2.70 | NH₂ | Dynabeads M-270 Amine | Polystyrene with magnetic material | - | Invitrogen |

The sedimentation pattern of the different types of particles tested was determined using alternatively three types of 96-well microtiter plates (VWR):
- Flat- bottom (Ref. NUNC269787).
- U-bottom (Ref. NUNC262162).
- V-bottom (Ref. NUNC249662).

Phosphate-buffered saline buffer (PBS) 10x (Ref. BP3994, Fisher Scientific) was diluted as indicated by the provider to produce 1x PBS (11.9 mM sodium phosphate, NaCl 137 mM, KCI 2.7 mM, pH 7.4). Agglutination of amine bearing particles was induced with bis[sulfosuccinimidyl] suberate (BS3; Ref. PG82083, Thermo Fisher). For the agglutination of carboxyl particles, 1-ethyl-3-(-3- dimethylaminopropyl) carbodiimide hydrochloride (EDC; Pierce Ref. PG82073) plus Poly(ethylene glycol) diamine (NH₂-PEG-NH₂, average number of monomers of 2000; Sigma Ref 753084) were used.

### Procedure:

Microparticles were washed once with PBS, were resuspended in PBS and were serially diluted (between 1:2 and 1:128) in the wells of a microtiter plate in either PBS or PBS supplemented with an agglutinating agent (BS3 or EDC + NH₂-PEG-NH₂). The plate was covered and was incubated for 30 min at room temperature (RT), while images of the sedimentation pattern were being registered every 5 min.

### Results:

As can be seen in FIG. 1, those beads with a particle core diameter from 3 to 10 µm and a material mass density of said core from 1.5 to 2.5 g/cc changed their sedimentation pattern upon agglutination. Indeed, they did not sediment when agglutinated.

Of the beads studied with these parametric values, Sicastar- blue 5 µm and 10 µm in diameter were of interest, since the changes in sedimentation patterns were bead concentration-dependent. In the absence of any agglutinating agent, 12,5 µL/well of 5 µm Sicastar-blue sedimented forming a halo at the bottom of the well. The presence of a chemical crosslinker (BS3) prevented bead sedimentation and the solution remained turbulent (agglutination). A slightly different effect was observed for 10 µm Sicastar-blue, which displayed a tighter sediment when non-agglutinated than in the presence of BS3, but only if 25-50 µL/well were used.

Sicastar-blue 5 µm and 10 µm were additionally tested in 3 types of 96-well microtiter plates, which had flat, U-shaped or V-shaped wells.

Both types of beads settled down forming a tight button at V-shaped wells, a behaviour that reminded that of nucleated avian erythrocytes. In contrast, the sediments in U-shaped wells were broader and, in the case of the 5 µm beads, formed a ring. Mammal erythrocytes, which are enucleated, are known to form ring-like sediments in U-shaped wells. The differences in sedimentation of 5 µm particles were only evident at U-shaped wells. In contrast, the differences in sedimentation displayed by 10 µm microparticles, agglutinated or not, were observable at both U- and V-shaped wells, although they were slightly clearer in V-shaped microtiter plates. No distinction could be made between free and agglutinated beads in flat-bottom plates in none of the cases.

As it is shown in FIG. 2, the beads selected sediment in about 5 min. The sedimentation pattern produced by agglutinated and non-agglutinated beads was stable over time and did not change if the incubation (and thus bead sedimentation time) was extended up to 45 min. According to these results, a 5-min sedimentation time was enough to differentiate by the naked eye between agglutinated and not agglutinated beads.

### Example 2. Method for producing particles modified with a sialylated bioreceptor

### Materials:

Glutaraldehyde (Ref. G6257), MES hydrate (Ref. M2933), and bovine serum albumin (BSA Ref. A7030) were obtained from Sigma-Aldrich (Madrid, Spain). 1-ethyl-3-(-3-dimethylaminopropyl) carbodiimide hydrochloride (EDC; Ref. PG82073) was acquired from Fisher Scientific (Madrid, Spain). Phosphate-buffered saline buffer 10x (Ref. BP3994, Fisher Scientific) was diluted as indicated by the provider to produce 1x PBS (11.9 mM sodium phosphate, NaCl 137 mM, KCI 2.7 mM, pH 7.4). TRIS Buffered Saline (TBS; 25 mM Tris, 140 mM sodium chloride, and 3.0 mM potassium chloride) was prepared using tablets from VWR Life Science (Ref. 97063-680). The blocking buffer consisted of TBS supplemented with 0.5% BSA.

Four highly silalylated proteins (porcine mucin Ref. M2378 (PM), bovine mucin Ref. M3895 (BM), glycophorin Ref. G5017 (GYPA), and avidin), and neutravidin, a deglycosylated protein used as a negative control, were obtained from Sigma-Aldrich.

### Procedure:

Unless otherwise stated, all incubations were carried out in Eppendorf tubes, at RT, protected from light and under end-to-end mixing using a Mini LabRoller rotator (Labnet International).

For particles displaying amine groups on surface,12.5 mg of silica microparticles were centrifuged for 3 min at 100 xG and the supernatant was discarded. The beads were next washed 3 times with PBS and were incubated in 0.5 mL of 8% glutaraldehyde for 30 min. Glutaraldehyde-activated microparticles were washed twice with PBS to remove the excess of glutaraldehyde, and were incubated for 2 h in 0.5 mL of PBS containing 150 µg of sialylated protein at 950 rpm in a Thermal Shake Lite (VWR). Protein-conjugated particles were then centrifuged and were incubated for 30 min with blocking buffer. The resulting microparticles were washed once more with PBS and were finally stored in PBS, 0.1% BSA at 4°C. For long-term storage, 0.1% of sodium azide (Ref.190381000, Acros Organics) was added.

For particles displaying carboxyl groups on surface, 25 mg of COOH-microspheres were washed 2 times with 1 mL of MES 0.5 M. The pellet was resuspended in 500 µL of MES, a freshly prepared mixture of EDC and NHS (4 mg of EDC and 8 mg of NHS dissolved in 125 µL) was added and the microparticles were incubated for 45 min with continuous end-to-end mixing. EDC-activated particles were washed once with PBS 1X and 200 µg of (sialylated) protein dissolved in PBS were added. After incubating for 3 h under continuous mixing, protein-conjugated particles were centrifuged and washed once with PBS. Then, 1 mL of blocking buffer was added and incubated 30 min. Finally, the conjugated microparticles were washed 3 times with PBS and were stored in PBS, 0,1% BSA at 4°C.

### Example 3. Effect of sialylated bioreceptor isoelectric point (Ip) and buffer pH on bead sedimentation

FIG. 3 shows the sedimentation pattern displayed by Sicastar blue beads (10 µm in ø), modified with different sialylated proteins and allowed to settle down while suspended in buffers of different pH. As it can be observed, the isoelectric point (Ip) of the sialylated protein incorporated on the bead surface conditioned its sedimentation pattern. Accordingly, medium pH had a profound effect on bead sedimentation. In general, the beads sedimented only in buffers of pH higher than the Ip of the sialylated protein conjugated on their surface. For instance, Sicastar blue beads (10 µm in ø) modified with MP or with MB (Ip in the range of 3-4) sedimented only at pHs above 3-4, while the same beads modified with GYPA (Ip around 6) formed buttoned sediments only in buffers of pH equal or higher than 6.

### Example 4. Agglutination assays for lectin detection

### Materials and procedure:

Sicastar-blue beads, 5 and 10 µm, were chemically modified with PM, BM, GYPA and avidin using alternatively glutaraldehyde or EDC. The modification protocol finished with extensive washing and a blocking step in order to remove poorly bound molecules, inactivate any remaining crosslinker reactive groups, cover the beads' surface, and prevent subsequent non-specific binding.

Lectins that displayed differential affinity for sialic acids were used as models to simulate the agglutination induced by influenza virus and optimize bead conjugation with bioreceptor candidates. Lectin from *Triticum vulgaris* (Ref. L9640, Sigma) has affinity mainly for N-acetyl-β-D- glucosamine oligomers, although it also recognizes Neu5Ac. In contrast, lectin from *Sambuccus nigra* (Ref. L6890, Sigma) interacts mostly with Neu5Acα2-6Gal(NAc), the same receptor as human influenza viruses.

Lectin 10-fold dilutions were added to the plate (50 µL per well in PBS). After that, 7.5 µL of conjugated-microparticles were added to the wells to reach a final volume of 100 µL. The plate was mixed by pipetting, was covered and was incubated for 15 min.

### Results:

The difference in sedimentation pattern between free (non-agglutinated) and agglutinated beads was studied. Two plant lectins, which are carbohydrate-binding proteins, were used for this purpose. *Sambucus nigra* agglutinin (SNA) is known to bind α-NeuNAc-[2→6]-Gal, α-NeuNAc-[2→6]-GalNAc, and, to a lesser extent, α-NeuNAc-[2→3]-Gal residues. Wheat germ agglutinin (WGA) from *Triticum vulgaris* displays affinity for N-acetyl-β-D-glucosaminyl residues and N-acetyl-β-D-glucosamine oligomers.

In these experiments, a fixed concentration of each type of conjugated microparticle was allowed to settle down for 10 min in the presence of increasing concentrations of lectin or no lectin at all. In all cases, the presence of increasing concentrations of lectin promoted agglutination (FIG. 4A-B) and prevented bead sedimentation at the bottom of recipient.

The change in bead sedimentation pattern was lectin-dependent, showing that they were induced by lectin binding and not randomly. For instance, while GYPA beads detected lower concentrations of SNA than of WGA, the beads modified with BM and PM were more sensitive for WGA (FIG. 4A-B). A similar pattern was observed for both 5 and 10 µm particles, but bigger particles seemed more sensitive and detected lectin concentrations 10 times lower.

The microscopic characterization revealed that while the beads remained individualized in PBS, the incubation in the presence of lectin resulted in the formation of aggregates (agglutinated beads) (FIG. 4C-E). The size of these aggregates increased proportionally to the concentration of lectin present. These results confirmed that the changes in sedimentation observed were caused by lectin-mediated agglutination.

### Example 5. Effect of bead size in the agglutination assays

For a fixed bead density, Sicastar blue beads of increasing diameter (i.e., ø 3-15 µm) displayed higher sedimentation speeds. This had an important effect in the sedimentation pattern of free beads and how sensitive they were for the detection of agglutinating lectins, and affected also bead manipulation.

FIG. 5A shows that the stock suspensions of protein-coated Sicastar blue 15 µm in diameter started sedimenting after mixing and were completely settled down in less than 3 min. This forced the user to resuspend the bead suspension each time before pipetting. In contrast, Sicastar blue 10 µm stock suspensions sedimented in about 5 min, Sicastar blue 5 µm needed approximately 15 min, and Sicastar blue 3 µm bead suspensions were not completely settled down until 1 hour later.

FIG. 5B shows that this affected the sedimentation pattern of both agglutinated and not agglutinated beads. Sicastar blue 15 µm did not form detectable buttons at the bottom of the wells, either when agglutinated or free. This was attributed to a too fast sedimentation, which could scatter the beads over the well bottom, but was not studied additionally. For Sicastar 3-10 µm, all formed observable buttons on the wells when free. In the three cases, the presence of an agglutinating lectin prevented sedimentation. However, bigger beads changed their sedimentation at lower lectin concentrations and were thus more sensitive to them. For instance, while Sicastar blue 3 µm changed their sedimentation in the presence of lectin concentrations above 5 µg/mL, Sicastar blue 5 µm detected lectin above 0.5 µg/mL and Sicastar blue 10 µm displayed changes in sedimentation in the presence of 0.05 µg/mL of lectin.

According to these results, for this bead density, bead sizes ranging 5-10 µm displayed the best performance.

### Example 6. Effect of bead density in the agglutination assays

Bead density was also determinant in the sedimentation of free and agglutinated beads.

As illustrated in FIG. 6, for a bead size around 3 µm (diameter range 1.5-3.11 µm), beads displaying density lower than 1.5 g/ccm or higher than 2.5 g/ccm did not form evident buttoned sediments on the well bottom. Accordingly, the presence of agglutinating agents did not produce an evident change in the sedimentation pattern. For instance, ProMag HP3 beads (ø 3.11 µm and density 1.4 g/ccm) displayed similar milky appearance in the absence and in the presence of agglutinating agents. On the other hand, the beads displaying densities in the limit of the tolerable range (such as ProMag 3 and Sicastar M, which exhibit densities of 1.6 and 2.5 g/ccm, respectively), produced faintly observable sediments free in solution, and although the difference in sedimentation produced by agglutinating agents was also weak, the two differential sedimentation patters could be observed. In contrast, and as optimum, Sicastar blue beads (in the image, ø 3 µm and density 1.8 g/ccm) produced clear buttoned sediments in the well when free, that vanished in the presence of agglutinating agents.

According to these results, bead densities ranging between 1.5 g/ccm and 2.5 g/ccm were needed in order to monitor changes in bead sedimentation caused by the presence of agglutinating agents.

### Example 7. Agglutination assays for influenza virus detection

Influenza virus interacts with erythrocytes though the HA of the viral envelop, which binds the sialic acids exposed on the cell surface. This produces erythrocyte agglutination and changes in sedimentation. It is well known that avian and mammal viruses recognize preferentially sialic acids interacting with galactose through α(2→3) and α(2→6) bounds, respectively. Here, GYPA, a highly sialylated protein obtained from the membrane of human erythrocytes, was used to bind influenza viruses. Neutravidin, a de-glycosilated protein, was used in parallel as a negative control.

For these experiments, 5 strains of inactivated influenza viruses were obtained from Hytest (Turku, Finland):
- Influenza A/New Caledonia/20/99 (H1N1, Ref. 81N73-3)
- Influenza A/Beijing/262/95 (H1N1, Ref. 81N73-2)
- Influenza A/Panama/2007/99 (H3N2, Ref. 8IN74-1)
- Influenza A/Brisbane/10/07 (H3N2, Ref. 8IN74-4)
- Influenza B/Victoria/504/00 (Ref. 8IN75-3)

FIG. 7 (A) displays the results obtained after mixing GYPA-particles (produced using Sicastar blue 10 µm in diameter as in Example 2) with increasing concentrations of H1N1 influenza virus (A/New Caledonia/20/99) in U-shaped microtiter plates, and allowing the mixtures to settle down for 5 min. As it is shown, GYPA-particles changed their sedimentation pattern in the presence of A(H1N1) virus down to 0.32 µg/mL. The figure shows that particles do not agglutinate and appear as a sedimented pellet on the bottom of the U-shaped well (as a dark hollowed circle) in the absence of virus, while they agglutinate in the presence of the virus (fade coloration among the U-shaped wells). As before, the microscopy study confirmed that GYPA agglutinated in the presence of influenza (FIG. 7C), compared to bead dispersion in the absence of the virus (FIG. 7B).

The behaviour of GYPA-particles was studied further in the presence of the other four viral strains (A/Beijing, A/Panama, A/Brisbane and influenza B), as depicted in FIG. 7D. In this case, the figure shows virus dilution instead of µg/mL, because hemagglutination titter is the unit regularly used to perform erythrocyte titrations in classical hemoagglutination assays. The minimal dilution that causes agglutination (the agglutination titter for that strain under to experimental conditions tested) is labelled in the figure by a vertical bar. As it can be observed, GYPA-particles recognized influenza A/Beijing (H1N1), although less sensitively than influenza A/New Caledonia (H1N1) (1:4 and 1:32 titters, respectively). GYPA-particles recognized the strain of influenza B virus tested as well (titter 1:8). In contrast, these particles did not recognize the two A(H3N2) strains tested (A/Panama and A/Brisbane).

FIG. 8 shows that carrying a pre-incubation of the GYPA-particles with the virus did not improve assay performance. On the contrary, GYPA-particles that had been preincubated in a tube for 90 min with increasing concentrations of influenza virus under agitation conditions, and had been then transferred to the wells of a microtiter plate, did sediment very similarly to free GYPA-particles in the absence of virus. This was attributed to the dynamic and faint binding produced by influenza HA/NA, in opposition to more stable binding displayed by bioreceptors such as antibodies.

The specificity of the recognition was established by performing a competitive assay with soluble GYPA added to the medium (FIG. 9). In the presence of high concentrations of GYPA in solution, the free protein competed with the bead-bound counterpart for the interaction of influenza virus. This resulted in virus kidnaping and inhibited the viral-induced agglutination, which was visible by the naked eye. In contrast, in the presence of other proteins that displayed no affinity for the virus, such as neutravidin or non-neutralizing Ab, no inhibition of the agglutination was observed (data not shown).

The images in FIGs 7-9 illustrate that the particles do really act as fresh RBCs in a hemagglutination-like assay. Therefore, the particles of the invention can be considered "synthetic erythrocytes" or "erythrocyte-mimicking reagents", also called herewith synthrocytes.

### Example 8. Agglutination inhibition assays

Finally, GYPA-particles (produced using Sicastar blue 10 µm in diameter as in Example 2) were used to carry out a serological agglutination inhibition assay. In this case, the experiment entailed adding a fixed amount of GYPA-particles to the U-shaped wells, which contained a fixed amount of virus (4 agglutination titters of influenza A/New Caledonia (H1N1) or no virus at all (negative control) plus increasing concentrations of an anti-influenza A H1N1 antibody.

Two mouse monoclonal antibodies were obtained from Antibodies online and were tested in parallel:
- Anti-influenza A hemagglutinin H1 antibody (clone 34-55, Ref. ABIN1605067), produced specifically against influenza A/NewCaledonia/20/99 (Ab1 in FIG. 10);
- Anti influenza A Virus H1N1 antibody (Ref. ABIN284533) produced against influenza A Virus H1N1 (A/Taiwan/1/86) and recommended for hemagglutination inhibition assays (Ab2 in FIG. 10);

FIG. 10 shows that, as expected, GYPA-particles sedimented in the absence of virus and antibody ("No virus" in the figure). In the presence of virus and without antibody in the medium (concentration of antibody "0" in the figure) or with concentrations of antibody below 1 µg/mL, GYPA-particles agglutinated and remained suspended. However, as the concentration of antibody raised above 1 µg/mL, the antibody bound to the virus and prevented virus binding to the beads, inhibiting particle agglutination. It is worth noting that the effect was more pronounced for Ab1 than for Ab2, which had been raised specifically for the virus strain that was used in these experiments.

As can be deduced from FIG. 10, the particles described herein have a differential sedimentation pattern when agglutinated (due to the presence of a ligand) *versus* the sedimentation pattern when non-agglutinated (when no ligand is present or a competitive ligand is added in the media, such as Ab1, Ab2, or serum containing antibodies from a subject).

Thus, with the particles described herein, agglutination assays and agglutination inhibition assays can be carried out, which can be operated similarly to classical methods such as the hemagglutination and hemagglutination inhibition assays (HAI), but produce results faster. Using synthetic erythrocytes instead of animal RBCs is compatible with the incorporation of selected bioreceptors or bioreceptor batteries, which should allow detecting specific analytes, a challenging task when using animal erythrocytes that cannot be modified easily. These assays would be convenient for testing the antigenic features of vaccine strain candidates or viral isolates produced by cell culture or embryonated eggs, or to monitor over time the integrity/stability of viral preparations (viral titration).

If competitors, such as Abs, are added, the antigenic characterization of a viral isolate can be done as well in order to determine the virus type, subtype and/or antigenic reference strain. Alternatively, the particles described above and the method of the invention would be useful to study the immunological response by neutralizing antibody titration in a serum sample, for instance to study vaccine immunization efficacy, to determine individual response after exposure to a pathogen, or to monitor population immunological response over an epidemic or pandemic outbreak.

In this way, producing a battery of particles displaying different viral receptors would permit performing the multiplex antigenic screening of specimens.

Example 9. Comparative performance of agglutination and agglutination inhibition assays using animal erythrocytes and GYPA-particles.

The performance of the particles and method of the invention were compared to those displayed by guinea pig erythrocytes in classical agglutination and HAI assays.

FIG. 11A displays the results obtained in parallel when testing a fixed amount of either guinea pig erythrocytes (top) or GYPA-particles (bottom; produced using Sicastar blue 10 µm in diameter as in Example 2) in the presence of increasing concentrations of influenza A/New Caledonia (H1N1) virus. As it can be observed, guinea pig erythrocytes were more sensitive, exhibiting changes in the sedimentation pattern for virus dilutions down to 1:64, while GYPA-particles were sensitive to the virus presence only for dilutions up to 1:16. In contrast, whereas guinea pig erythrocytes had to be incubated for 60 min to display their characteristic sedimentation pattern, GYPA-particles provided results in <5 min.

In the same way, an agglutination inhibition assay performed in parallel with guinea pig erythrocytes and GYPA-particles displayed comparable results. As before, GYPA-particles were faintly less sensitive, but significantly faster (FIG. 11B).

Example 10. Agglutination inhibition assays performed with the erythrocytes-mimicking synthrocytes (left, 5 min) of the invention or human RBCs (right, 60 min), using reference virus and antisera (previously treated with RDE) provided by the WHO, through the Centers for Disease Control and Prevention (CDC, Atlanta, USA).

Finally, the performance of the particles described above and the method of the invention were evaluated using the reagents kit provided by the WHO-CDC for influenza surveillance (campaign 2019), following when possible the protocols and experimental conditions recommended by the WHO.

FIG. 12 displays the results obtained in parallel when testing a fixed amount of either human erythrocytes or GYPA-particles (produced using Sicastar blue 10 µm in diameter as in Example 2) in the presence of fixed concentrations of either influenza AH1N1pdm09, B/Victoria or B/Yamagata virus, and decreasing concentrations of the corresponding positive and negative control antiserums. In all cases, the presence of antibodies specific against a viral strain prevented its binding to RBCs and synthrocytes, inhibiting their agglutination. On the contrary, the incubation with negative control antisera (that did not contain specific antibodies) did not prevent agglutination. As before, GYPA-particles were faintly less sensitive than RBCs, but still displayed differential agglutination in the presence of positive and negative antisera, and were significantly faster.

### Citation List

- WO201133450 (Mesoscale technologies LLC).
- Hosaka et al., "Preparation of microspheres of poly(glycidyl methacrylate) and its derivatives as carriers for immobilized proteins" Immunological communications - 1980, vol. no. 12(5), pp.: 509-517.
- Patent US5405784A of 11th April 1995 (Chemunex, Maisons Alfort, France).
- Patent US4355102A of 19th October 1982 (Institut National de la Sante et de la Recherche Medicale, INSERM).

## Claims

1. A method for detecting and/or characterizing pathogens present in an isolated test sample, said method comprising:
(a) contacting in a vessel comprising a bottom and a wall, and in a buffered aqueous media, the sample where pathogens are to be detected, with a coloured particle comprising:
- a core with a surface, said core having a material mass density from 1.5 to 2.5 g/cc; and a diameter from 3 to 10 µm; and wherein at least the surface is coloured and it comprises one or more functional groups selected from -COO-R¹, -NR² R³, and combinations thereof, wherein R¹ is independently selected from H, and (C₁-C₈)-alkyl, and R² and R³ are independently selected from H, (C₁-C₈)-alkyl, and (C₁-C₈)-alkoxyl; and
- a pathogen ligand that is a compound comprising a pathogen-recognizing molecular pattern immobilized on the surface of the core;
(b) allowing the particles to sediment for a time from 1 to 20 minutes after the contacting step;
(c) determining the sedimentation pattern of the particles, wherein particle sedimentation on the bottom or walls of the vessel indicates lack of pathogen in that sample, while the lack or absence of sedimentation is indicative of agglutinated particles, which means that interaction between the pathogen ligand on the surface of the particle and the pathogen in the sample has taken place and that the pathogen has been recognized by pathogen ligand,
wherein step (a) is performed at a pH that is higher than the average isoelectric point of the particle, said average isoelectric point provided by the surface electrical charge of pathogen ligand immobilized on the surface of the core of the particle and the functional groups selected from -COOR1, -NR2R3 also on the said core surface of the particle.

2. The method according to claim 1, wherein the particle core has a material mass density from 1.5 to 2.0 g/cc; and a diameter from 3 to 10 µm.

3. The method according to any one of claims 1-2, wherein the particle core and its surface is made of a material selected from the group consisting of glass, silica, sand, wood, fiber, natural polymer, synthetic polymer, and combinations thereof.

4. The method according to any one of claims 1-3, wherein the pathogen ligand is a glycoprotein.

5. The method according to claim 4, wherein the pathogen ligand is a sialylated protein or sialylated glycoprotein.

6. The method according to claim 5, wherein the sialylated protein is a sialylglycoprotein selected from glycophorin, podocalyxin, mucin, fetuin, transferrin, gangliosides, luteinizing hormone (LH), follicle-stimulating hormone (FSH), human chorionic gonadotropin (hCG), and combinations thereof.

7. The method according to claim 1, which comprises isolated or synthetic sialic acids immobilized on the core surface.

8. The method according to any one of claims 1-7, wherein in step (a) a competitive ligand of the pathogen is added, competing for the interaction of the pathogen ligand on the surface of the particle with the pathogen in the sample.

9. A method for detecting and/or characterizing pathogen-binding antibodies present in an isolated test sample, said method comprising:
(a) contacting in a vessel comprising a bottom and a wall, and in a buffered aqueous media, the sample where antibodies are to be detected with a pathogen, and with a coloured particle as defined in any one of claims 1-7;
(b) allowing the particles to sediment for a time from 1 to 20 minutes after the contacting step;
(c) determining the sedimentation pattern of the particles, wherein particle sedimentation on the bottom or walls of the vessel indicates presence of pathogen-binding antibodies in that sample, which antibodies inhibited pathogen binding to the particles and particle agglutination,
wherein step (a) is performed at a pH that is higher than the average isoelectric point of the particle, said average isoelectric point provided by the surface electrical charge of pathogen ligand immobilized on the surface of the core of the particle and the functional groups selected from -COOR1, -NR2R3 also on the said core surface of the particle.

10. The method according to any one of claims 1-9, wherein the pathogen is selected from the group consisting of influenza A virus, influenza B virus, influenza C virus, coronavirus, measles virus, Newcastle Disease virus, hepatitis virus, rhinoviruses, adenoviruses, human parainfluenza viruses, human respiratory syncytial virus, enteroviruses, metapneumovirus, reovirus, rotavirus, bluetongue virus.

11. The method according to any one of claims 1-10, wherein the pathogen is influenza virus and the coloured particle is a particle made of silica and comprises immobilized on its surface a sialylated protein as a pathogen ligand.

12. Use of a kit for carrying out a method as defined in any one of claims 1-7 for detecting and optionally antigenically characterizing pathogens comprised in an isolated test sample, said kit comprising the coloured particle as defined in any one of claims 1-7, and one or more vessels with a V-shaped or U-shape bottom, and instructions and means to carry out the method.

13. Use of the coloured particle as defined in any one of claims 1-7, as erythrocyte-mimicking reagent, wherein the erythrocyte-mimicking reagent is a reagent for an hemagglutination-like assay selected from an hemagglutination (HA) assay and a hemagglutination inhibition (HAI) assay.

## Patentansprüche

1. Ein Verfahren zum Nachweis und/oder zur Charakterisierung von Krankheitserregern, die in einer isolierten Testprobe vorhanden sind, wobei das Verfahren Folgendes umfasst:
(a) In-Kontakt-bringen der Probe, in der Krankheitserreger nachgewiesen werden sollen, mit einem gefärbten Partikel in einem Behälter, der einen Boden und eine Wand umfasst, und in einem gepufferten wässrigen Medium
umfassend:
- einen Kern mit einer Oberfläche, wobei der Kern eine Materialmassendichte von 1,5 bis 2,5 g/cm³ und einen Durchmesser von 3 bis 10 µm hat; und wobei mindestens die Oberfläche gefärbt ist und eine oder mehrere funktionelle Gruppen umfasst, die ausgewählt sind aus -COO-R¹, -NR²R³ und Kombinationen davon, wobei R¹ unabhängig ausgewählt ist aus H und (C₁-C₈)-Alkyl und R² und R³ unabhängig voneinander ausgewählt sind aus H, (C₁-C₈)-Alkyl und (C₁-C₈)-Alkoxy; und
- einen Krankheitserregerliganden, der eine Verbindung ist, die ein Krankheitserreger erkennendes molekulares Muster umfasst, das auf der Oberfläche des Kerns immobilisiert ist;
(b) die Partikel für eine Zeit von 1 bis 20 Minuten nach dem Kontaktschritt sedimentieren lassen;
(c) Bestimmen des Sedimentationsmusters der Partikel, wobei die Partikelsedimentation auf dem Boden oder den Wänden des Behälters auf ein Fehlen am Krankheitserreger in dieser Probe hinweist, während das Fehlen oder Nichtvorhandensein der Sedimentation auf agglutinierte Partikel hinweist, was bedeutet, dass eine Wechselwirkung zwischen dem Krankheitserregerliganden auf der Oberfläche des Partikels und dem Krankheitserreger in der Probe stattgefunden hat und dass der Krankheitserreger von dem Krankheitserregerliganden erkannt wurde,
wobei Schritt (a) bei einem pH-Wert durchgeführt wird, der höher als der durchschnittliche isoelektrische Punkt des Partikels ist, wobei der durchschnittliche isoelektrische Punkt durch die elektrische Oberflächenladung des Krankheitserregerliganden, der auf der Oberfläche des Kerns des Partikels immobilisiert ist, und die funktionellen Gruppen, die aus -COOR1, -NR2R3 ausgewählt sind, ebenso auf der Kernoberfläche des Partikels, gegeben wird.

2. Das Verfahren nach Anspruch 1, wobei der Partikelkern eine Materialmassendichte von 1,5 bis 2,0 g/cm³ und einen Durchmesser von 3 bis 10 µm hat.

3. Das Verfahren nach einem der Ansprüche 1 bis 2, wobei der Partikelkern und seine Oberfläche aus einem Material hergestellt sind, das aus der Gruppe ausgewählt ist, die aus Glas, Siliciumdioxid, Sand, Holz, Fasern, natürlichem Polymer, synthetischem Polymer und Kombinationen davon besteht.

4. Das Verfahren nach einem der Ansprüche 1- 3, wobei der Krankheitserregerligand ein Glykoprotein ist.

5. Das Verfahren nach Anspruch 4, wobei der Krankheitserregerligand ein sialyliertes Protein oder sialyliertes Glykoprotein ist.

6. Das Verfahren nach Anspruch 5, wobei das sialylierte Protein ein Sialylglycoprotein ist, das ausgewählt ist aus Glycophorin, Podocalyxin, Mucin, Fetuin, Transferrin, Gangliosiden, luteinisierendem Hormon (LH), follikelstimulierendem Hormon (FSH), humanem Choriongonadotropin (hCG) und Kombinationen davon.

7. Das Verfahren nach Anspruch 1, welches isolierte oder synthetische Sialinsäuren umfasst, die auf der Kernoberfläche immobilisiert sind.

8. Das Verfahren nach einem der Ansprüche 1 bis 7, wobei in Schritt (a) ein kompetitiver Ligand des Krankheitserregers hinzugefügt wird, der um die Wechselwirkung des Krankheitserregerliganden auf der Oberfläche des Partikels mit dem Krankheitserreger in der Probe konkurriert.

9. Ein Verfahren zum Nachweis und/oder zur Charakterisierung von an Krankheitserreger bindenden Antikörpern, die in einer isolierten Testprobe vorhanden sind, wobei das Verfahren Folgendes umfasst:
(a) In-Kontakt-bringen, in einem Behälter, der einen Boden und eine Wand umfasst, und in einem gepufferten wässrigen Medium, der Probe, in der Antikörper nachgewiesen werden sollen, mit einem Krankheitserreger und mit einem gefärbten Partikel wie in einem der Ansprüche 1 bis 7 definiert;
(b) die Partikel für eine Zeit von 1 bis 20 Minuten nach dem Kontaktschritt sedimentieren lassen;
(c) Bestimmen des Sedimentationsmusters der Partikel, wobei die Partikelsedimentation auf dem Boden oder den Wänden des Behälters auf das Vorhandensein von an Krankheitserreger bindenden Antikörpern in dieser Probe hinweist, wobei die Antikörper die Krankheitserregerbindung an die Partikel und die Partikelagglutination inhibierten, wobei Schritt (a) bei einem pH-Wert durchgeführt wird, der höher als der durchschnittliche isoelektrische Punkt des Partikels ist, wobei der durchschnittliche isoelektrische Punkt durch die elektrische Oberflächenladung des Krankheitserregerliganden, der auf der Oberfläche des Kerns des Partikels immobilisiert ist, und die funktionellen Gruppen, die aus -COOR1, -NR2R3 ausgewählt sind, ebenso auf der Kernoberfläche des Partikels, gegeben wird.

10. Das Verfahren nach einem der Ansprüche 1 bis 9, wobei der Krankheitserreger ausgewählt ist aus der Gruppe bestehend aus Influenza A-Virus, Influenza B-Virus, Influenza C-Virus, Coronavirus, Masernvirus, Newcastle-Krankheit-Virus, Hepatitis-Virus, Rhinoviren, Adenoviren, humanen Parainfluenzaviren, humanen respiratorischen Synzytialviren, Enteroviren, Metapneumoviren, Reovirus, Rotavirus, Blauzungenvirus.

11. Das Verfahren nach einem der Ansprüche 1 bis 10, wobei der Krankheitserreger das Influenzavirus ist und das gefärbte Partikel ein Partikel aus Siliziumdioxid ist und auf seiner Oberfläche immobilisiert ein sialyliertes Protein als Krankheitserregerligand umfasst.

12. Verwendung eines Kits zur Durchführung eines Verfahrens wie in einem der Ansprüche 1 bis 7 definiert zum Nachweis und gegebenenfalls zur antigenen Charakterisierung von Krankheitserregern, die in einer isolierten Testprobe enthalten sind, wobei das Kit das gefärbte Partikel wie in einem der Ansprüche 1 bis 7 definiert und ein oder mehrere Behälter mit einem V-förmigen oder U-förmigen Boden sowie Anweisungen und Mittel zur Durchführung des Verfahrens umfasst.

13. Verwendung des gefärbten Partikels wie in einem der Ansprüche 1 bis 7 definiert, als Erythrozyten nachahmendes Reagenz, wobei das Erythrozyten nachahmende Reagenz ein Reagenz für einen Hämagglutinations ähnlichen Test ist, der aus einem Hämagglutinationstest (HA, *hemagglutination* assay) und einem Hämagglutinationshemmtest (HAI, *hemagglutination inhibition* assay) ausgewählt ist.

## Revendications

1. Un procédé de détection et/ou de caractérisation d'agents pathogènes présents dans un échantillon de test isolé, ledit procédé comprenant :
(a) mettre en contact dans un récipient comprenant un fond et une paroi, et dans un milieu aqueux tamponné, l'échantillon où des agents pathogènes doivent être détectés, avec une particule colorée
comprenant :
- un noyau avec une surface, ledit noyau ayant une densité de masse de matériau allant de 1,5 à 2,5 g/cm³ ; et un diamètre allant de 3 à 10 µm ; et dans lequel au moins la surface est colorée et elle comprend un ou plusieurs groupes fonctionnels choisis parmi - COO-R¹, -NR²R³, et des combinaisons de ceux-ci, où R¹ est indépendamment choisi parmi H, et alkyle en (C₁-C₈), et R² et R³ sont indépendamment choisis parmi H, alkyle en (C₁-C₈), et alcoxyle en (C₁-C₈) ; et
- un ligand d'agent pathogène qui est un composé comprenant un motif moléculaire de reconnaissance d'agent pathogène immobilisé à la surface du noyau ;
(b) laisser les particules sédimenter pendant un temps allant de 1 à 20 minutes après l'étape de mise en contact ;
(c) déterminer le modèle de sédimentation des particules, dans lequel la sédimentation des particules sur le fond ou les parois du récipient indique l'absence d'agent pathogène dans cet échantillon, tandis que la manque ou l'absence de sédimentation est indicative de particules agglutinées, ce qui signifie que l'interaction entre le ligand de l'agent pathogène à la surface de la particule et l'agent pathogène dans l'échantillon a eu lieu et que l'agent pathogène a été reconnu par le ligand de l'agent pathogène,
dans lequel l'étape (a) est réalisée à un pH qui est supérieur au point isoélectrique moyen de la particule, ledit point isoélectrique moyen étant donné par la charge électrique de surface du ligand d'agent pathogène immobilisé sur la surface du noyau de la particule et les groupes fonctionnels choisis parmi -COOR1, -NR2R3 également sur ladite surface de noyau de la particule.

2. Le procédé selon la revendication 1, dans lequel le noyau de particule a une densité de masse de matériau de 1,5 à 2,0 g/cm³ ; et un diamètre de 3 à 10 µm.

3. Le procédé selon l'une quelconque des revendications 1 à 2, dans lequel le noyau de particule et sa surface sont constitués d'un matériau choisi dans le groupe constitué par le verre, la silice, le sable, le bois, les fibres, du polymère naturel, du polymère synthétique et des combinaisons de ceux-ci.

4. Le procédé selon l'une quelconque des revendications 1 à 3, dans lequel le ligand d'agent pathogène est une glycoprotéine.

5. Le procédé selon la revendication 4, dans lequel le ligand d'agent pathogène est une protéine sialylée ou une glycoprotéine sialylée.

6. Le procédé selon la revendication 5, dans lequel la protéine sialylée est une sialylglycoprotéine choisie parmi la glycophorine, la podocalyxine, la mucine, la fétuine, la transferrine, les gangliosides, l'hormone lutéinisante (LH), l'hormone folliculo-stimulante (FSH), la gonadotropine chorionique humaine (hCG) et des combinaisons de celles-ci.

7. Le procédé selon la revendication 1, qui comprend des acides sialiques isolés ou synthétiques immobilisés sur la surface du noyau.

8. Le procédé selon l'une quelconque des revendications 1 à 7, dans lequel, dans l'étape (a), un ligand compétitif de l'agent pathogène est ajouté, en compétition pour l'interaction du ligand d'agent pathogène à la surface de la particule avec l'agent pathogène dans l'échantillon.

9. Un procédé de détection et/ou de caractérisation d'anticorps de liaison à agent pathogène présents dans un échantillon de test isolé, ledit procédé comprenant :
(a) mettre en contact dans un récipient comprenant un fond et une paroi, et dans un milieu aqueux tamponné, l'échantillon où les anticorps doivent être détectés avec un agent pathogène, et avec une particule colorée telle que défini dans l'une quelconque des revendications 1 à 7 ;
(b) laisser les particules sédimenter pendant un temps allant de 1 à 20 minutes après l'étape de mise en contact ;
(c) déterminer le modèle de sédimentation des particules, dans lequel la sédimentation des particules sur le fond ou les parois du récipient indique la présence d'anticorps de liaison à agent pathogène dans cet échantillon, lesquels anticorps ont inhibé la liaison de l'agent pathogène aux particules et l'agglutination des particules,
dans lequel l'étape (a) est réalisée à un pH qui est supérieur au point isoélectrique moyen de la particule, ledit point isoélectrique moyen étant donné par la charge électrique de surface du ligand d'agent pathogène immobilisé sur la surface du noyau de la particule et les groupes fonctionnels choisis parmi -COOR1, -NR2R3, également sur ladite surface de noyau de la particule.

10. Le procédé selon l'une quelconque des revendications 1 à 9, dans lequel l'agent pathogène est choisi dans le groupe constitué par le virus de la grippe A, le virus de la grippe B, le virus de la grippe C, le coronavirus, le virus de la rougeole, le virus de la maladie de Newcastle, le virus de l'hépatite, les rhinovirus, les adénovirus, les virus parainfluenza humains, le virus syncytial respiratoire humain, les entérovirus, le métapneumovirus, le réovirus, le rotavirus, le virus de la fièvre catarrhale du mouton (virus bluetongue).

11. Le procédé selon l'une quelconque des revendications 1 à 10, dans lequel l'agent pathogène est le virus de la grippe et la particule colorée est une particule faite en silice et comprend, immobilisée sur sa surface, une protéine sialylée en tant que ligand de liaison à agent pathogène.

12. Utilisation d'un kit pour la mise en œuvre d'un procédé tel que défini dans l'une quelconque des revendications 1 à 7 pour la détection et facultativement la caractérisation antigénique d'agents pathogènes compris dans un échantillon de test isolé, ledit kit comprenant la particule colorée telle que définie dans l'une quelconque des revendications 1 à 7, et un ou plusieurs récipients avec un fond en forme de V ou en forme de U, et des instructions et des moyens pour mettre en œuvre le procédé.

13. Utilisation de la particule colorée telle que définie dans l'une quelconque des revendications 1 à 7, en tant que réactif imitant les érythrocytes, dans laquelle le réactif imitant les érythrocytes est un réactif pour un test de type hémagglutination choisi parmi un test d'hémagglutination (HA, *hemagglutination* assay) et un test d'inhibition de l'hémagglutination (HAI, *hemagglutination inhibition* assay).
